# EUROPEAN PATENT SPECIFICATION

(11) **EP 1 130 022 B1**
(45) Date of publication and mention of the grant of the patent: **22.07.2009**
(21) Application number: 00104135.9
(22) Date of filing: 29.02.2000
(51) Int. Cl.: C07D 498/08

(54) **3-Aza-6,8-dioxabicyclo[3.2.1]octanes and analogues and combinatorial libraries containing them**
3-Aza-6,8-dioxabicyclo[3.2.1]octane und Analoga sowie diese enthaltende kombinatorische Bibliotheken
3-Aza-6,8-dioxabicyclo[3.2.1]octanes et d'analogues et bibliothèques combinatoires les contenant

(43) Date of publication of application: 05.09.2001
(73) Proprietor: Universita Degli Studi di Firenze, 50121 Florence (IT)
(72) Inventor: Guarna, Antonio, 59011 Seano (Province of Prato) (IT); Menchi, Gloria, 50019 Sesto Fiorentino (Prov. Florence) (IT); Occhiato, Ernesto Giovanni, 50142 Firenze (IT); Machetti, Fabrizio, 59100 Prato (IT); Scarpi, Dina, 50134 Firenze (IT)
(74) Representative: Gervasi, Gemma

(56) References cited:
- BE-A- 892 853
- J.-Q. WANG, W.-S. TIAN: J. CHEM. SOC. PERKIN TRANS. 1, no. 2, 1996, pages 209-212, XP002142445
- A. GUARNA ET AL.: "Synthesis and Reactivity of Bicycles Derived from Tartaric Acid and alpha-Amino Acids: A Novel Class of Conformationally Constrained Dipeptide Isosteres Based upon Enantiopure 3-Aza-6,8-dioxabicyclo[3.2.1]octane-7-carb oxylic Acid" J. ORG. CHEM., vol. 64, no. 20, 1999, pages 7347-7364, XP002142446

## Description

### Field of the invention

The present invention refers to heterobicycle derivatives of general formula (I) 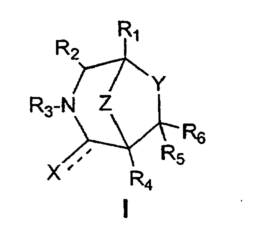 wherein:
R₁, is chosen in the group consisting of C₁₋₈alkyl, C₂₋₈alkenyl, C₂₋₈alkinyl, cycloalkyl, aryl, heterocycle, arylC₁₋₈alkyl; heterocycleC₁₋₈alkyl; RR'N-C₁₋₈alkyl, RR'N-aryl, RO-aryl, R(O)C-aryl, RO(O)C-aryl, RR'N(O)C-aryl, (P)-W-NR-aryl, (P)-W-O-aryl, (P)-W-C(O)O-aryl, (P)-W-O(O)C-aryl, (P)-W-C(O)RN-aryl, (P)-W-NR(O)C-aryl;
R₂, is chosen in the group consisting of H, C₁₋₈alkyl, C₂₋₈alkenyl, C₂₋₈alkinyl, cycloalkyl, aryl, arylC₁₋₈alkyl; heterocycleC₁₋₈alkyl; aminoC₁₋₈alkyl, aminoaryl, C₁₋₈ alkyloxyaryl, hydroxyaryl, carboxyaryl, carboalkyloxyaryl, alkylcarbamoylaryl,-(side chain), -(side chain)-W-(P) or
R₁ and R₂ taken together are a C₁₋₄alkyl, C₂₋₄alkenyl, cycloalkyl, benzofused cycloalkyl, to form a bridge of 3, 4, 5, 6 terms;
R₃, is chosen in the group consisting H, C₁₋₈alkyl, C₂₋₈alkenyl, C₂₋₈alkinyl, cycloalkyl, aryl, arylC₁₋₈alkyl; heterocycleC₁₋₈alkyl; RR'NC₁₋₈alkyl, RR'Naryl, RO-C₁₋₈alkyl, RO(O)C-C₁₋₈alkyl, R(O)C-C₁₋₈alkyl, RC(O)O-C₁₋₈alkyl, RC(O)N(R)C₁₋₈alkyl RO-aryl, RO(O)C-aryl, R(O)C-aryl RC(O)O-aryl, RC(O)N(R)aryl, -CH(amino acid side-chain)CO₂R, -CH(amino acid side-chain)C(O)NR, -CH(amino acid side-chain)-C(O)O-W-(P), -CH(amino acid side-chain)-C(O)N(R)-W-(P), CH(CO₂R)-amino acid side-chain-W-(P), CH(CONRR')-amino acid side-chain-W-(P), protecting group;
R₄ and R₅, same or different, are chosen in the group consisting H, C₁₋₈alkyl, C₂₋₈ alkenyl, C₂₋₈alkinyl, cycloalkyl, aryl, heterocycle, arylC₁₋₈alkyl; heterocycleC₁₋₈alkyl;
R₆ is chosen in the group consisting, H, C₁₋₈alkyl, C₂₋₈alkenyl, C₂₋₈alkinyl, cycloalkyl, aryl, arylC₁₋₈alkyl, heterocycle, heterocycleC₁₋₈alkyl; -C(O)R, -C(O)OR, -C(O)NRR', CH₂OR, CH₂NRR', -C(O)NH-CH(amino acid side-chain)C(O)OR, - C(O)O-W-(P),-C(O)N(R)-W-(P), -CH₂O-W-(P), -CH₂N(R)-W-(P);
R and R', same or different, are chosen in the group consisting of: H, C₁₋₈alkyl, C₂₋₈alkenyl, C₂₋₈alkinyl, cycloalkyl, aryl, heterocycle, arylC₁₋₈alkyl; heterocycleC₁₋₈ alkyl; a protecting group, -C(O)CH-(amino acid side-chain)-NHR, -NH-CH(amino acid side-chain)COOR, -CH(amino acid side-chain)COOR;
P is resin, both soluble or bound to a solid support;
W is as linker;
X is O, S, when a is a double bond, or X is H and a is single bond.
Y and Z, same or different, are O, S, SO, SO₂, N-R, wherein R is as above defined;
wherein the term cycloalkyl represents: cyclopropane, cyclobutane, cyclopentane, cyclohexane, cycloheptane, cyclooctane, norbornane, canphane, adamantine; the term aryl specifies phenyl, biphenyl and naphtyl groups substituted with one or two moieties chosen from the groups consisting of halogen, cyano, nitro, C₁₋₆ alkyl; the term heterocycle represents pyridine, imidazole, pyrrole, indole, triazoles, pyrrolidine, pyperidine and the term protecting group refers to known groups capable of preventing reaction or bonding of oxygen, nitrogen, carboxylic acids, thiols, alcohols, amines;
the above said alkyl-, alkenyl-, alkinyl-, cycloalkyl-, aryl- and heterocycle-groups, being possibly substituted with the proviso that:
when: Z = Y = X = O, R₂= R₄ = R₅ = H, R₃ = CH₃ and R₆ = Ph then R₁=is not Ph; and
when X = R₂ = H and Y = Z = O then at least one R₄, R₅ or R₆ is not H.

Compounds of formula I and their libraries are useful to discover new leads for therapeutical applications.

### State of the art

The process of discovering new therapeutically active compounds involves the screening of a large number of compounds, in order to develop a structure-activity relationships and select the structures which could represent a new lead for the biological target. Fast methods are necessary to prepare a large collection of compounds to submit to the screening and this, in recent years, can be achieved by preparation of combinatorial chemical libraries of well designed chemical compounds by using immobilization techniques on soluble or insoluble resins. Heterocycles compounds, bearing different substituents, and functionalised with reactive groups suitable for anchoring on resins, are very useful for this new type of synthetic strategy (for example see US 5,925,527). Another important point for a well designed chemical library is the complete control of the configuration of the sterogenic centers and the possibility to have enantiopure compounds. All these above mentioned features can be incorporated in compounds of general formula (I) which can be obtained with only two reaction steps starting from easily prepared precursors, available also as pure enantiomers. This new type of compounds, having a rigid bicyclic structure, can be functionalised in several positions and allows the easy anchoring on resin support, thus representing a new scaffold for the generation of combinatorial libraries. Thus compounds of general formula (I) can be used for the discover of new leads for therapeutical applications.

In BE 892.853 some compounds of formula (I) are described and their analgesic properties are reported.

Analogously in J. Chem. Soc. Perkin Trans. 1, no 2, 1996, pages 209-12 a compound of formula (I) is described (see page 210 compound 12) as an intermediate compound in the synthesis of neoclausenamide.

Compounds of general formula (I) having R₁ = H, Y and Z = O, have been already prepared as it is described by us in JOC 1999, 64, 7347 by a process involving various steps starting from a suitable α-amino alcohol which is coupled with a tartaric acid derivative. The prepared intermediate required an oxidation of the primary alcohol function to the corresponding aldehyde and a subsequent trans-acetalization to arrive to compounds I having R₁ = H and X,Y and Z = O. However, it can be seen that the above described process involves many steps which can have a negative effect on the final yields of the desired compounds and the application cannot be extended to compound having R₁ different from H, and Z and Y different from O. Moreover this above described process is limited because, involving also an oxidative step, is compatible only with the functions resistant to the oxidative conditions and requires the protection of the all function sensitive to oxidation.

Therefore the application refers to a new straightforward process which, in only two steps, can produce compounds I, where R₁ is different from H, by starting from α-aminoketone II 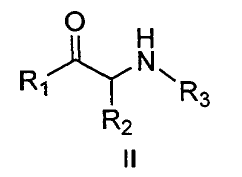 and acid derivative III, 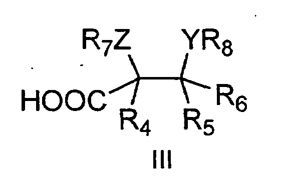 commercially available or easily prepared by reported procedures. Moreover, this procedure, allowing the immobilization of each the precursors II or III to a soluble or insoluble resin support, is suitable for the synthesis of combinatorial chemical libraries (see for examples J Med Chem 1999, 42, 3743; US 5,958,792, US 5,302,589) either as separate synthesis, in mixture synthesis, split and recombine synthesis, parallel synthesis with manual or automated fashion.

### Detailed description of the invention

The present invention allows to overcome the above said problems thanks to the compounds of formula (I) as above defined useful either as individual compounds or for generation of combinatorial chemical libraries either in mixture synthesis or parallel synthesis with manual or automated fashion.

Moreover the invention refers to a new an advantageous process for the preparation of the above defined compounds of formula (I) and their use for discovering new leads for therapeutical applications.

According to the present invention in the compounds of formula (I) as above defined:
Resin (P) means any polymeric material either soluble in the solvents commonly used in organic synthesis or bound to the solid support;
Solid support is any solid material (at room temperature) to which starting resin materials (reactive groups) may be bound;
W is any molecule which can be used as linker to bound the resin P to the reagents and the products of formula (I);

Protecting group means any group capable of preventing the atom to which it is attached from participating in an undesired reaction or bonding, as commonly used in synthesis reactions.

Amino acid side-chain means the side chain moieties of the natural occurring L or D amino acids or the non naturally occurring amino acids;

More preferably the resin is a polymeric material derivatised with a reactive group such as, for example, a -NH₂ group or other electron donating group such as an hydroxyl group.

Preferred solid support materials comprise polymeric compounds such as polyethylene and polystyrene compounds and related inert polymeric compounds. The substrate may be in any shape including sheets, the inside of a cylindrical vessel, or pins but are preferably in the form of spherical beads less than 1.0 cm in diameter more preferably less than 1.0 mm in diameter. A "substrate" or solid support is a conventional solid support material used in peptide synthesis. Non-limiting examples of such substrates or supports include a variety of support resins and connectors to the support resins such as those which are photocleavable, DKP-forming linkers (DKP is diketopiperazine; see, e.g., WO90/09395 incorporated herein by reference), TFA cleavable, HF cleavable, fluoride ion cleavable, reductively cleavable and base-labile linkers. A solid support resin comprises a plurality of solid support particles which can be split into portions for separate reactions and recombined as desired. Preferred protecting groups are those which prevent reaction or bonding of oxygen, nitrogen, carboxylic acids, thiols, alcohols, amines and the like. Such groups and their preparation and introduction are conventional in the art and include, for example, for the reactive function OH: benzyl, *tert*-butyl; acetals, esters, trialkylsilylethers; for COOH group: methyl, tert-butyl, benzyl, allyl esters; for the NH group: t-Boc, Fmoc, CBz, Bn, Bz.

Amino acid side-chain means the different amino acid side-chain moieties attached to an "amino acid". The term "amino acid" includes any one of the twenty L or D natural α-amino acids having as " side chain":, -H of glycine; -CH₃ of alanine; -CH(CH₃)₂ of valine; -CH₂CH(CH₃)₂ of leucine; -CH(CH₃)CH₂CH₃ of isoleucine; -CH₂OH of serine; -CH(OH)CH₃ of threonine; --CH₂SH of cysteine;-CH₂CH₂SCH₃ of methionine; -CH₂-(phenyl) of phenylalanine; -CH₂-(phenyl)-OH of tyrosine; -CH₂-(indole group) of tryptophan; -CH₂ COOH of aspartic acid;-CH₂C(O)(NH₂) of asparagine; -CH₂CH₂COOH of glutamic acid; - CH₂CH₂C(O)NH₂ of glutamine; -CH₂CH₂CH₂-N(H)C(NH₂)NH of arginine; -CH₂-(imidazole) group of histidine; and -CH₂(CH₂)₃NH₂ of lysine, comprising the same amino acid side-chain moieties bearing suitable protecting groups (Pg). In addition, the term "amino acid" include also non naturally occurring amino acids, like norleucine (Nle), norvaline (NVa), β-alanine, L or D α-phenyl glycine and others well known in the peptide art.

In the compounds of formula (I), as above defined, groups C₁₋₈ alkyl, C₂₋₈ alkenyl and C₂₋₈ alkinyl represent linear or branched alkyl radicals as for example: methyl, ethyl, propyl, isopropyl, butyl, pentyl, hexyl, heptyl, octyl, ethylene, propene, butene, isobutene, acetylene, propine, butine etc

The term cycloalkyl represents: cyclopropane, cyclobutane, cyclopentane, cyclohexane, cycloheptane, cyclooctane, norbornane, canphane, adamantane. The term aryl specifies phenyl, biphenyl and naphtyl groups substituted with one or more, and preferably one or two moieties chosen from the groups consisting of halogen, cyano, nitro, C₁₋₆ alkyl. The term heterocycle represents in particular: saturated or aromatic heterocycles containing one or more N atoms, more particularly: pyridine, imidazole, pyrrole, indole, triazoles, pyrrolidine, pyperidine.

The term halogen represent fluorine, chlorine, bromine, iodine.

The terms "library", "combinatorial library", "resin-derived library" and the like are used interchangeably throughout the description to mean a series of separate individual components or mixture of the compounds I, synthesized in solution or on a solid support from one or more solid phase bound resin starting materials. and their pharmaceutically acceptable salts or esters.

The synthetic process according to the invention involves only two steps and moreover uses, as starting compounds, an α-aminoketone and a carboxylic acid derivative bearing two vicinal nucleophilic groups like OH, SH, or NHR, preferably belonging to the classes of α,β-dihydroxy acid or α-amino-β-hydroxy acid or α-hydroxy-β-amino acid or α,β-dithiol acid derivatives.

In particular, the process according to the present invention allows the preparation of the compounds of formula (I) wherein:
a = double bond, and X = O or a = single bond and X = H
Y and Z, same or different are O, S, NR wherein R is above described
R₁ = methyl, ethyl, propyl, isopropyl, t-butyl, benzyl, phenyl, 4-hydrophenyl, 4-methoxy-phenyl, 4-carboxy-phenyl, 4-nitro-phenyl, 4-amino-phenyl, 4-halogen-phenyl, 4-trifluoromethylphenyl, 2-hydrophenyl, 2-methoxy-phenyl, 2-carboxy-phenyl, 2-nitro-phenyl, 2-amino-phenyl, 2-halogen-phenyl, 2-trifluoromethylphenyl C₁₋₈alkylOC(O)phenyl, hydroxy-C₁₋₈alkylphenyl, methoxy-C₁₋₈alkylphenyl, RR'NC(O)-phenyl, RR'N-C₁₋₈alkylphenyl, biphenyl, naphtyl, tetrahydronapthyl, decahydronaphtyl, cycloalkyl, heterocycle, (P)-W-NR-phenyl, (P)-W-O-phenyl, (P)-W-C(O)O-phenyl, (P)-W-O(O)C-phenyl, (P)-W-C(O)RN-phenyl, (P)-W-NR(O)C-phenyl, wherein (P), W, R and R' are defined as above;
R₂, which can be bound with R₁ through a C₁-C₅alkyl chain, is chosen in the group consisting of H, methyl, ethyl, propyl, isopropyl, t-butyl, phenyl, 4-hydrophenyl, 4-methoxy-phenyl, 4-carboxy-phenyl, 4-amino-phenyl, benzyl, amino acid side chain-; (P)-W-amino acid side-chain;
R₃, H, methyl, ethyl, propyl, isopropyl, t-butyl, phenyl, benzyl, cycloalkyl, aryl, arylC₁₋₈alkyl; heterocycle, heterocycleC₁₋₈alkyl-CH(amino acid side-chain)CO₂R, CH(amino acid side-chain)C(O)NR, -CH(amino acid side-chain)-C(O)O-W-(P),-CH(amino acid side-chain)-C(O)N(R)-W-(P), CH(CO₂R)-amino acid side-chain-W-(P), CH(CONRR')- amino acid side-chain-W-(P), Pg, wherein (P), (amino acid side-chain), W, R and R' are defined as above;
R₄, R₅, same or different, are chosen in the group consisting H, methyl, ethyl, propyl, isopropyl, phenyl, benzyl, heterocycle
R₆ is chosen in the group consisting, H, methyl, ethyl, propyl, isopropyl, t-butyl, phenyl, benzyl, cycloalkyl, aryl, benzyl, heterocycle, heterocycleC₁₋₈alkyl; COOH, COOR, C(O)R, CONHR CONRR', CH₂OH, CH₂OR CH₂NHR, CH₂NRR', -C(O)NH-CH(amino acid side-chain)C(O)OR, -C(O)O-W-(P), -C(O)N(R)-W-(P), -CH₂O-W-(P), -CH₂N(R)-W-(P), wherein R and R' same or different and the terms "(amino acid side-chain)", "(P)", and "W" are as above defined

Among the pharmaceutically acceptable esters and salts according to the present invention the following can be mentioned: hydrochloride, sulfate, citrate, formiate, phosphate.

According to the invention the above defined compounds of formula (I) can be prepared starting from compounds of general formula II 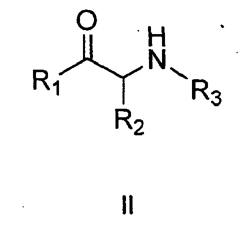 wherein R₁, R₂, R₃, are as above defined
and III 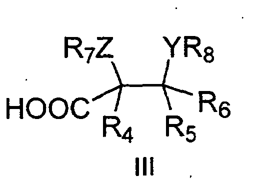 wherein R₄, R₅, R₆, Y and Z are as above defined,
and R₇ R₈ represent H or suitable protecting groups, (Pg) which can be same or different, cyclic or acyclic, and which can be cleaved in acidic conditions.

The α-amino ketones II are commercially available or can be prepared as shown in the scheme 2, for example starting from an α-halogen-ketone V and a primary amine VI according to known procedures (see for example Tetrahedron Letters 1987, 28, 1287 and references cited therein)

The acid derivatives III are commercial available o can be prepared according know procedures.

As it can be seen from the Scheme 1 the preparation of the compounds (I) according to the invention involves, in the Step 1, the reaction of the α-amino ketone II with the acid derivative III to give the amide derivative IV in the presence of a coupling reagent. Because Step I involves the formation of an amide bond , all the reagents commonly used for the peptide synthesis can be applied to this step. Preferably the reaction is performed in an aprotic polar solvent, preferably CH₂Cl₂ or DMF, at a temperature comprised between 0°C - 100°C, preferably at 25°C, for a time comprised between 1 and 24 hours, preferably in the presence of a coupling agent and activator of the carboxy group, as PyBrOP, PyBOP, HATU, HOBt, HBTU, TBTU, DCC, DIC, EDC etc. and a tertiary base as NEt₃, DIPEA, NMM.

The intermediate amide IV is then cyclized into the final compound I in the Step 2, by action of an acid, which, allows the ketalization of the functions Z and Y with the carbonyl group by also removing the protecting groups Pg, when present. Also for this step the reaction conditions (temperature and time) and the type of acid and solvent are important.

The best results were obtained using a stechiometric or preferably catalytic amount of a strong acid, preferably sulphuric acid adsorbed on silica gel, p-toluen sulphonic acid, trifluoroacetic acid, trifluorometansulphonic acid and performing the reaction at a temperature comprised between 0°C - 150°C, preferably at refluxing-solvent temperature, in an organic apolar solvent (for example methylene chloride, chloroform, benzene or toluene) for a time comprised between 15 min and 24 hours, preferably 30 min -2 hours, preferably with the simultaneous removal of a portion of the solvent and eventually in the presence of molecular sieves. In these condition the final product I is obtained having X=O and *a* double bond. The subsequent reaction on the amide bond either with usual reducing agents, for example LiAlH₄, BH₃.THF, BH₃.Me₂S and like, produce compounds I wherein X=H and a is single bond, or by the use of sulphurating agents, like the Lawesson reagent, produce compounds I wherein X=S and *a* is double bond. Owing to the importance to produce combinatorial chemical libraries, the above reported procedure can be modified by using one of the two components II and III of the Step 1 bound to a resin through a suitable linker. In this case, the formed compound IV is also bound to a resin, and the following step 2 can be performed either maintaining the final product I bound to the resin or with a simultaneous cleavage from the resin. Because the starting α-amino ketone II can be easily prepared from an α-halogen ketone V and a primary amine VI (as reported in the Scheme 2), this can increase the molecular diversity of compounds II, by starting from of one of the two components V or VI, already immobilized on the resin-support.

Specific compounds I prepared according to the process of the invention are reported in the following table:

| Comp. | X | Z | Y | R₁ | R₂ | R₃ | R₄ | R₅ | R₆ |
|---|---|---|---|---|---|---|---|---|---|
| 1. | O | O | O | Ph | H | PhCH₂ | H | H | COOH |
| 2. | O | O | O | 4-HO-Ph | H | PhCH₂ | H | H | COOH |
| 3. | O | O | O | 4-O₂N-Ph | H | PhCH₂ | H | H | COOH |
| 4. | O | O | O | 4-H₂N-Ph | H | PhCH₂ | H | H | COOH |
| 5. | O | O | O | 4-MeO(O)C-Ph | H | PhCH₂ | H | H | COOH |
| 6. | O | O | O | 4-Me-Ph | H | PhCH₂ | H | H | COOH |
| 7. | O | O | O | 4-MeO-Ph | H | PhCH₂ | H | H | COOH |
| 8. | O | O | O | 4-Cl-Ph | H | PhCH₂ | H | H | COOH |
| 9. | O | O | O | 4-Br-Ph | H | PhCH₂ | H | H | COOH |
| 10. | O | O | O | 2-HO-Ph | H | PhCH₂ | H | H | COOH |
| 11. | O | O | O | 2-O₂N-Ph | H | PhCH₂ | H | H | COOH |
| 12. | O | O | O | 2-H₂N-Ph | H | PhCH₂ | H | H | COOH |
| 13. | O | O | O | 2-MeO(O)C-Ph | H | PhCH₂ | H | H | COOH |
| 14. | O | O | O | 2-Me-Ph | H | PhCH₂ | H | H | COOH |
| 15. | O | O | O | 2-MeO-Ph | H | PhCH₂ | H | H | COOH |
| 16. | O | O | O | 2-Cl-Ph | H | PhCH₂ | H | H | COOH |
| 17. | O | O | O | 2-Br-Ph | H | PhCH₂ | H | H | COOH |
| 18. | O | O | O | 2-Nafthyl | H | PhCH₂ | H | H | COOH |
| 19. | O | O | O | 2-thienyl | H | PhCH₂ | H | H | COOH |
| 20. | O | O | O | 4-biphenyl | H | PhCH₂ | H | H | COOH |
| 21. | O | O | O | Ph | H | Me | H | H | COOH |
| 22. | O | O | O | Ph | H | CH₃(CH₂)₂ | H | H | COOH |
| 23. | O | O | O | Ph | H | cyclohexyl | H | H | COOH |
| 24. | O | O | O | Ph | H | allyl | H | H | COOH |
| 25. | O | O | O | Ph | H | Ph | H | H | COOH |
| 26. | O | O | O | Ph | H | 4-HO-Ph | H | H | COOH |
| 27. | O | O | O | Ph | H | 4-O₂N-Ph | H | H | COOH |
| 28. | O | O | O | Ph | H | 4-MeO₂C-Ph | H | H | COOH |
| 29. | O | O | O | Ph | H | 4-Me-Ph | H | H | COOH |
| 30. | O | O | O | Ph | H | 4-MeO-Ph | H | H | COOH |
| 31. | O | O | O | Ph | H | 4-Cl-Ph | H | H | COOH |
| 32. | O | O | O | Ph | H | 4-Br-Ph | H | H | COOH |
| 33. | O | O | O | Ph | H | 2-HO-Ph | H | H | COOH |
| 34. | O | O | O | Ph | H | 2-O₂N-Ph | H | H | COOH |
| 35. | O | O | O | Ph | H | 2-MeO₂C-Ph | H | H | COOH |
| 36. | O | O | O | Ph | H | 2-Me-Ph | H | H | COOH |
| 37. | O | O | O | Ph | H | 2-MeO-Ph | H | H | COOH |
| 38. | O | O | O | Ph | H | 2-Cl-Ph | H | H | COOH |
| 39. | O | O | O | Ph | H | 2-Br-Ph | H | H | COOH |
| 40. | O | O | O | Ph | H | 2-Nafthyl | H | H | COOH |
| 41. | O | O | O | Ph | H | 2-thienyl | H | H | COOH |
| 42. | O | O | O | Ph | H | 4-biphenyl | H | H | COOH |
| 43. | O | O | O | Ph | H | 4-MeO₂C-PhCH₂ | H | H | COOH |
| 44. | O | O | O | Ph | H | 4-Me-PhCH₂ | H | H | COOH |
| 45. | O | O | O | Ph | H | 4-MeOPhCH₂ | H | H | COOH |
| 46. | O | O | O | Ph | H | 4-Cl-PhCH₂ | H | H | COOH |
| 47. | O | O | O | Ph | H | 4-Br-PhCH₂ | H | H | COOH |
| 48. | O | O | O | Ph | H | 2-HO-PhCH₂ | H | H | COOH |
| 49. | O | O | O | Ph | H | 2-O₂N-PhCH₂ | H | H | COOH |
| 50. | O | O | O | Ph | H | 2-MeO₂C-PhCH₂ | H | H | COOH |
| 51. | O | O | O | Ph | H | 2-Me-PhCH₂ | H | H | COOH |
| 52. | O | O | O | Ph | H | 2-MeO-PhCH₂ | H | H | COOH |
| 53. | O | O | O | Ph | H | 2-Cl-PhCH₂ | H | H | COOH |
| 54. | O | O | O | Ph | H | 2-Br-PhCH₂ | H | H | COOH |
| 55: | O | O | O | 4-HO-Ph | H | 4-HO-Ph CH₂ | H | H | COOH |
| 56. | O | O | O | 4-HO-Ph | H | 4-O₂N-PhCH₂ | H | H | COOH |
| 57. | O | O | O | 4-HO-Ph | H | 4-MeO₂C-PhCH₂ | H | H | COOH |
| 58. | O | O | O | 4-HO-Ph | H | 4-Me-PhCH₂ | H | H | COOH |
| 59. | O | O | O | 4-HO-Ph | H | 4-MeOPhCH₂ | H | H | COOH |
| 60. | O | O | O | 4-HO-Ph | H | 4-Cl-PhCH₂ | H | H | COOH |
| 61. | O | O | O | 4-HO-Ph | H | 4-Br-PhCH₂ | H | H | COOH |
| 62. | O | O | O | 4-HO-Ph | H | 2-HO-PhCH₂ | H | H | COOH |
| 63. | O | O | O | 4-HO-Ph | H | 2-O₂N-PhCH₂ | H | H | COOH |
| 64. | O | O | O | 4-HO-Ph | H | 2-MeO₂C-PhCH₂ | H | H | COOH |
| 65. | O | O | O | 4-HO-Ph | H | 2-Me-PhCH₂ | H | H | COOH |
| 66. | O | O | O | 4-HO-Ph | H | 2-MeO-PhCH₂ | H | H | COOH |
| 67. | O | O | O | 4-HO-Ph | H | 2-Cl-PhCH₂ | H | H | COOH |
| 68. | O | O | O | 4-HO-Ph | H | 2-Br-PhCH₂ | H | H | COOH |
| 69. | O | O | O | 4-HO-Ph | H | Me | H | H | COOH |
| 70. | O | O | O | 4-HO-Ph | H | CH₃(CH₂)₂ | H | H | COOH |
| 71. | O | O | O | 4-HO-Ph | H | cyclohexyl | H | H | COOH |
| 72. | O | O | O | 4-HO-Ph | H | allyl | H | H | COOH |
| 73. | O | O | O | Ph | H | HO₂C-CH₂ | H | H | COOH |
| 74. | O | O | O | Ph | H | Bn(HO₂C)CH | H | H | COOH |
| 75. | O | O | O | Ph | H | HOCH₂(HO₂C)CH | H | H | COOH |
| 76. | O | O | O | Ph | H | CH₃(HO)CH(HO₂C)CH | H | H | COOH |
| 77. | O | O | O | Ph | H | MeS(CH₂)₂(HO₂C)CH | H | H | COOH |
| 78. | O | O | O | Ph | H | H₂N(CH₂)₃(HO₂C)CH | H | H | COOH |
| 79. | O | O | O | Ph | H | HO₂CCH₂(HO₂C)CH | H | H | COOH |
| 80. | O | O | O | Ph | H | imidazole-CH₂(HO₂C)CH | H | H | COOH |
| 81. | O | O | O | Ph | H | indole-CH₂(HO₂C)CH | H | | COOH |
| 82. | O | O | O | 4-HO-Ph | H | HO₂C-CH₂ | H | H | COOH |
| 83. | O | O | O | 4-HO-Ph | H | Me(HO₂C)CH | H | H | COOH |
| 84. | O | O | O | 4-HO-Ph | H | (CH₃)₂CH(HO₂C)CH | H | H | COOH |
| 85. | O | O | O | 4-HO-Ph | H | Bn(HO₂C)CH | H | H | COOH |
| 86. | O | O | O | 4-HO-Ph | H | HOCH₂(HO₂C)CH | H | H | COOH |
| 87. | O | O | O | 4-HO-Ph | H | CH₃(HO)CH(HO₂C)CH | H | H | COOH |
| 88. | O | O | O | 4-HO-Ph | H | MeS(CH₂)₂(HO₂C)CH | H | H | COOH |
| 89. | O | O | O | 4-HO-Ph | H | H₂N(CH₂)₃(HO₂C)CH | H | H | COOH |
| 90. | O | O | O | 4-HO-Ph | H | HO₂CCH₂(HO₂C)CH | H | H | COOH |
| 91. | O | O | O | 4-HO-Ph | H | i imidazole-CH₂(HO₂C)CH | H | H | COOH |
| 92. | O | O | O | 4-HO-Ph | H | indole-CH₂(HO₂C)CH | H | H | COOH |
| 93. | O | O | O | Ph | Me | PhCH₂ | H | H | COOH |
| 94. | O | O | O | 4-HO-Ph | Me | PhCH₂ | H | H | COOH |
| 95. | O | O | O | Ph | Bn | PhCH₂ | H | H | COOH |
| 96. | O | O | O | 4-HO-Ph | Bn | PhCH₂ | H | H | COOH |
| 97. | O | O | O | Ph | Me | HO₂C-CH₂ | H | H | COOH |
| 98. | O | O | O | 4-HO-Ph | Me | HO₂C-CH₂ | H | H | COOH |
| 99. | O | O | O | Ph | Bn | HO₂C-CH₂ | H | H | COOH |
| 100. | O | O | O | 4-HO-Ph | Bn | HO₂C-CH₂ | H | H | COOH |
| 101. | O | O | O | Ph | Me | Bn(HO₂C)CH | H | H | COOH |
| 102. | O | O | O | 4-HO-Ph | Me | Bn(HO₂C)CH | H | H | COOH |
| 103. | O | HN | O | Ph | H | PhCH₂ | H | H | CH₃ |
| 104. | O | HN | O | Ph | Me | PhCH₂ | H | H | CH₃ |
| 105. | O | HN | O | Ph | Bn | PhCH₂ | H | H | CH₃ |
| 106. | O | HN | O | 4-OH-Ph | H | PhCH₂ | H | H | CH₃ |
| 107. | O | HN | O | 4-OH-Ph | Me | PhCH₂ | H | H | CH₃ |
| 108. | O | HN | O | 4-OH-Ph | Bn | PhCH₂ | H | H | CH₃ |
| 109. | O | HN | O | Ph | H | Ph | H | H | CH₃ |
| 110. | O | HN | O | Ph | Me | Ph | H | H | CH₃ |
| 111. | O | HN | O | Ph | Bn | Ph | H | H | CH₃ |
| 112. | O | HN | O | 4-OH-Ph | H | Ph | H | H | CH₃ |
| 113. | O | HN | O | 4-OH-Ph | Me | Ph | H | H | CH₃ |
| 114. | O | HN | O | 4-OH-Ph | Bn | Ph | H | H | CH₃ |
| 115. | O | HN | O | Ph | H | CH₃-Ph | H | H | CH₃ |
| 116. | O | HN | O | Ph | Me | CH₃-Ph | H | H | CH₃ |
| 117. | O | HN | O | Ph | Bn | CH₃-Ph | H | H | CH₃ |
| 118. | O | HN | O | 4-OH-Ph | H | CH₃-Ph | H | H | CH₃ |
| 119. | O | HN | O | 4-OH-Ph | Me | CH₃-Ph | H | H | CH₃ |
| 120. | O | HN | O | 4-OH-Ph | Bn | CH₃-Ph | H | H | CH₃ |
| 121. | O | HN | O | Ph | H | 4-MeO-PhCH₂ | H | H | CH₃ |
| 122. | O | HN | O | Ph | Me | 4-MeO-PhCH₂ | H | H | CH₃ |
| 123. | O | HN | O | Ph | Bn | 4-MeO-PhCH₂ | H | H | CH₃ |
| 124. | O | HN | O | 4-OH-Ph | H | 4-MeO-PhCH₂ | H | H | CH₃ |
| 125. | O | HN | O | 4-OH-Ph | Me | 4-MeO-PhCH₂ | H | H | CH₃ |
| 126. | O | HN | O | 4-OH-Ph | Bn | 4-MeO-PhCH₂ | H | H | CH₃ |
| 127. | O | HN | O | Ph | H | CH₃-PhCH₂ | H | H | CH₃ |
| 128. | O | HN | O | Ph | Me | CH₃-PhCH₂ | H | H | CH₃ |
| 129. | O | HN | O | Ph | Bn | CH₃-PhCH₂ | H | H | CH₃ |
| 130. | O | HN | O | 4-OH-Ph | H | CH₃-PhCH₂ | H | H | CH₃ |
| 131. | O | HN | O | 4-OH-Ph | Me | CH₃-PhCH₂ | H | H | CH₃ |
| 132. | O | HN | O | 4-OH-Ph | Bn | CH₃-PhCH₂ | H | H | CH₃ |
| 133. | O | HN | O | Ph | H | Me | H | H | CH₃ |
| 134. | O | HN | O | Ph | H | CH₃(CH₂)₂ | H | H | CH₃ |
| 135. | O | HN | O | Ph | H | cyclohexyl | H | H | CH₃ |
| 136. | O | HN | O | Ph | H | alkyl | H | H | CH₃ |
| 137. | O | HN | O | 4-OH-Ph | H | Me | H | H | CH₃ |
| 138. | O | HN | O | 4-OH-Ph | H | CH₃(CH₂)₂ | H | H | CH₃ |
| 139. | O | HN | O | 4-OH-Ph | H | cyclohexyl | H | H | CH₃ |
| 140. | O | HN | O | 4-OH-Ph | H | allyl | H | H | CH₃ |
| 141. | O | HN | O | Ph | H | HO₂C-CH₂ | H | H | CH₃ |
| 142. | O | HN | O | Ph | Me | HO₂C-CH₂ | H | H | CH₃ |
| 143. | O | HN | O | Ph | Bn | HO₂C-CH₂ | H | H | CH₃ |
| 144. | O | HN | O | 4-OH-Ph | H | HO₂C-CH₂ | H | H | CH₃ |
| 145. | O | HN | O | 4-OH-Ph | Me | HO₂C-CH₂ | H | H | CH₃ |
| 146. | O | HN | O | 4-OH-Ph | Bn | HO₂C-CH₂ | H | H | CH₃ |
| 147. | O | HN | O | Ph | H | Bn(HO₂C)CH | H | H | CH₃ |
| 148. | O | HN | O | Ph | Me | Bn(HO₂C)CH | H | H | CH₃ |
| 149. | O | HN | O | Ph | Bn | Bn(HO₂C)CH | H | H | CH₃ |
| 150. | O | HN | O | 4-OH-Ph | H | Bn(HO₂C)CH | H | H | CH₃ |
| 151. | O | HN | O | 4-OH-Ph | Me | Bn(HO₂C)CH | H | H | CH₃ |
| 152. | O | HN | O | 4-OH-Ph | Bn | Bn(HO₂C)CH | H | H | CH₃ |
| 153. | H | O | O | Ph | H | PhCH₂ | H | H | COOH |
| 154. | H | O | O | Ph | Me | PhCH₂ | H | H | COOH |
| 155. | H | O | O | Ph | Bn | PhCH₂ | H | H | COOH |
| 156. | H | O | O | 4-HO-Ph | H | PhCH₂ | H | H | COOH |
| 157. | H | O | O | 4-HO-Ph | Me | PhCH₂ | H | H | COOH |
| 158. | H | O | O | 4-HO-Ph | Bn | PhCH₂ | H | H | COOH |
| 159. | H | O | O | Ph | H | HO₂C-CH₂ | H | H | COOH |
| 160. | H | O | O | Ph | Me | HO₂C-CH₂ | H | H | COOH |
| 161. | H | O | O | Ph | Bn | HO₂C-CH₂ | H | H | COOH |
| 162. | H | O | O | 4-HO-Ph | H | HO₂C-CH₂ | H | H | COOH |
| 163. | H | O | O | 4-HO-Ph | Me | HO₂C-CH₂ | H | H | COOH |
| 164. | H | O | O | 4-HO-Ph | Bn | HO₂C-CH₂ | H | H | COOH |
| 165. | H | O | O | Ph | H | Bn(HO₂C)CH | H | H | COOH |
| 166. | H | O | O | Ph | Me | Bn(HO₂C)CH | H | H | COOH |
| 167. | H | O | O | Ph | Bn | Bn(HO₂C)CH | H | H | COOH |
| 168. | H | O | O | 4-HO-Ph | H | Bn(HO₂C)CH | H | H | COOH |
| 169. | H | O | O | 4-HO-Ph | Me | Bn(HO₂C)CH | H | H | COOH |
| 170. | H | O | O | 4-HO-Ph | Bn | Bn(HO₂C)CH | H | H | COOH |
| 171. | H | HN | O | Ph | H | PhCH₂ | H | H | CH₃ |
| 172. | H | HN | O | Ph | H | 4-MeO-PhCH₂ | H | H | CH₃ |
| 173. | H | HN | O | Ph | Me | PhCH₂ | H | H | CH₃ |
| 174. | H | HN | O | Ph | Bn | PhCH₂ | H | H | CH₃ |
| 175. | H | HN | O | 4-OH-Ph | H | PhCH₂ | H | H | CH₃ |
| 176. | H | HN | O | 4-OH-Ph | Me | PhCH₂ | H | H | CH₃ |
| 177. | H | HN | O | 4-OH-Ph | Bn | PhCH₂ | H | H | CH₃ |
| 178. | H | HN | O | Ph | H | HO₂C-CH₂ | H | H | CH₃ |
| 179. | H | HN | O | Ph | Me | HO₂C-CH₂ | H | H | CH₃ |
| 180. | H | HN | O | Ph | Bn | HO₂C-CH₂ | H | H | CH₃ |
| 181. | H | HN | O | 4-OH-Ph | H | HO₂C-CH₂ | H | H | CH₃ |
| 182. | H | HN | O | 4-OH-Ph | Me | HO₂C-CH₂ | H | H | CH₃ |
| 183. | H | HN | O | 4-OH-Ph | Bn | HO₂C-CH₂ | H | H | CH₃ |
| 184. | H | HN | O | Ph | H | Bn(HO₂C)CH | H | H | CH₃ |
| 185. | H | HN | O | Ph | Me | Bn(HO₂C)CH | H | H | CH₃ |
| 186. | H | HN | O | Ph | Bn | Bn(HO₂C)CH | H | H | CH₃ |
| 187. | H | HN | O | 4-OH-Ph | H | Bn(HO₂C)CH | H | H | CH₃ |
| 188. | H | HN | O | 4-OH-Ph | Me | Bn(HO₂C)CH | H | H | CH₃ |
| 189. | H | HN | O | 4-OH-Ph | Bn | Bn(HO₂C)CH | H | H | CH₃ |
| 190. | H | HN | O | Ph | H | Ph | H | H | CH₃ |
| 191. | H | HN | O | Ph | Me | Ph | H | H | CH₃ |
| 192. | H | HN | O | Ph | Bn | Ph | H | H | CH₃ |
| 193. | H | HN | O | 4-OH-Ph | H | Ph | H | H | CH₃ |
| 194. | H | HN | O | 4-OH-Ph | Me | Ph | H | H | CH₃ |
| 195. | H | HN | O | 4-OH-Ph | Bn | Ph | H | H | CH₃ |
| 196. | H | HN | O | Ph | H | CH₃-Ph | H | H | CH₃ |
| 197. | H | HN | O | Ph | Me | CH₃-Ph | H | H | CH₃ |
| 198. | H | HN | O | Ph | Bn | CH₃-Ph | H | H | CH₃ |
| 199. | H | HN | O | 4-OH-Ph | H | CH₃-Ph | H | H | CH₃ |
| 200. | H | HN | O | 4-OH-Ph | Me | CH₃-Ph | H | H | CH₃ |
| 201. | H | HN | O | 4-OH-Ph | Bn | CH₃-Ph | H | H | CH₃ |
| 202. | H | HN | O | Ph | H | 4-MeO-PhCH₂ | H | H | CH₃ |
| 203. | H | HN | O | Ph | Me | 4-MeO-PhCH₂ | H | H | CH₃ |
| 204. | H | HN | O | Ph | Bn | 4-MeO-PhCH₂ | H | H | CH₃ |
| 205. | H | HN | O | 4-OH-Ph | H | 4-MeO-PhCH₂ | H | H | CH₃ |
| 206. | H | HN | O | 4-OH-Ph | Me | 4-MeO-PhCH₂ | H | H | CH₃ |
| 207. | H | HN | O | 4-OH-Ph | Bn | 4-MeO-PhCH₂ | H | H | CH₃ |
| 208. | H | HN | O | Ph | H | CH₃-PhCH₂ | H | H | CH₃ |
| 209. | H | HN | O | Ph | Me | CH₃-PhCH₂ | H | H | CH₃ |
| 210. | H | HN | O | Ph | Bn | CH₃-PhCH₂ | H | H | CH₃ |
| 211. | H | HN | O | 4-OH-Ph | H | CH₃-PhCH₂ | H | H | CH₃ |
| 212. | H | HN | O | 4-OH-Ph | Me | CH₃-PhCH₂ | H | H | CH₃ |
| 213. | H | HN | O | 4-OH-Ph | Bn | CH₃-PhCH₂ | H | H | CH₃ |
| 214. | H | O | O | Ph | H | PhCH₂ | H | H | CH₂OH |
| 215. | H | O | O | Ph | H | 4-MeOPhCH₂ | H | H | CH₂OH |
| 216. | H | O | O | Ph | Me | PhCH₂ | H | H | CH₂OH |
| 217. | H | O | O | Ph | Bn | PhCH₂ | H | H | CH₂OH |
| 218. | H | O | O | 4-HO-Ph | H | PhCH₂ | H | H | CH₂OH |
| 219. | H | O | O | 4-HO-Ph | Me | PhCH₂ | H | H | CH₂OH |
| 220. | H | O | O | 4-HO-Ph | Bn | PhCH₂ | H | H | CH₂OH |
| 221. | H | O | O | Ph | H | HOCH₂ | H | H | CH₂OH |
| 222. | H | O | O | Ph | Me | HOCH₂ | H | H | CH₂OH |
| 223. | H | O | O | Ph | Bn | HOCH₂ | H | H | CH₂OH |
| 224. | H | O | O | 4-HO-Ph | H | HOCH₂ | H | H | CH₂OH |
| 225. | H | O | O | 4-HO-Ph | Me | HOCH₂ | H | H | CH₂OH |
| 226. | H | O | O | 4-HO-Ph | Bn | HOCH₂ | H | H | CH₂OH |
| 227. | H | O | O | Ph | H | Bn(HOH₂C)CH | H | H | CH₂OH |
| 228. | H | O | O | Ph | Me | Bn(HOH₂C)CH | H | H | CH₂OH |
| 229. | H | O | O | Ph | Bn | Bn(HOH₂C)CH | H | H | CH₂OH |
| 230. | H | O | O | 4-HO-Ph | H | Bn(HOH₂C)CH | H | H | CH₂OH |
| 231. | H | O | O | 4-HO-Ph | Me | Bn(HOH₂C)CH | H | H | CH₂OH |
| 232. | H | O | O | 4-HO-Ph | Bn | Bn(HOH₂C)CH | H | H | CH₂OH |
| 233. | H | HN | O | Ph | H | PhCH₂ | H | H | H |
| 234. | H | HN | O | Ph | H | 4-MeO-PhCH₂ | H | H | H |
| 235. | H | HN | O | Ph | Me | PhCH₂ | H | H | H |
| 236. | H | HN | O | Ph | Bn | PhCH₂ | H | H | H |
| 237. | H | HN | O | 4-OH-Ph | H | PhCH₂ | H | H | H |
| 238. | H | HN | O | 4-OH-Ph | Me | PhCH₂ | H | H | H |
| 239. | H | HN | O | 4-OH-Ph | Bn | PhCH₂ | H | H | H |
| 240. | H | HN | O | Ph | H | HOCH₂ | H | H | H |
| 241. | H | HN | O | Ph | Me | HOOCH₂ | H | H | H |
| 242. | H | HN | O | Ph | Bn | HOCH₂ | H | H | H |
| 243. | H | HN | O | 4-OH-Ph | H | HOCH₂ | H | H | H |
| 244. | H | HN | O | 4-OH-Ph | Me | HOCH₂ | H | H | H |
| 245. | H | HN | O | 4-OH-Ph | Bn | HOCH₂ | H | H | H |
| 246. | H | HN | O | Ph | H | Bn(HOH₂C)CH | H | H | H |
| 247. | H | HN | O | Ph | Me | Bn(HOH₂C)CH | H | H | H |
| 248. | H | HN | O | Ph | Bn | Bn(HOH₂C)CH | H | H | H |
| 249. | H | HN | O | 4-OH-Ph | H | Bn(HOH₂C)CH | H | H | H |
| 250. | H | HN | O | 4-OH-Ph | Me | Bn(HOH₂C)CH | H | H | H |
| 251. | H | HN | S | Ph | H | PhCH₂ | H | H | H |
| 252. | H | HN | S | Ph | H | 4-MeO-PhCH₂ | H | H | H |
| 253. | H | HN | S | Ph | Me | PhCH₂ | H | H | H |
| 254. | H | HN | S | Ph | Bn | PhCH₂ | H | H | H |
| 255. | H | HN | S | 4-OH-Ph | H | PhCH₂ | H | H | H |
| 256. | H | HN | S | 4-OH-Ph | Me | PhCH₂ | H | H | H |
| 257. | H | HN | S | 4-OH-Ph | Bn | PhCH₂ | H | H | H |
| 258. | H | HN | S | Ph | H | HOCH₂ | H | H | H |
| 259. | H | HN | S | Ph | Me | HOCH₂ | H | H | H |
| 260. | H | HN | S | Ph | Bn | HOCH₂ | H | H | H |
| 261. | H | HN | S | 4-OH-Ph | H | HOCH₂ | H | H | H |
| 262. | H | HN | S | 4-OH-Ph | Me | HOCH₂ | H | H | H |
| 263. | H | HN | S | 4-OH-Ph | Bn | HOCH₂ | H | H | H |
| 264. | H | HN | S | Ph | H | Bn(HOH₂C)CH | H | H | H |
| 265. | H | HN | S | Ph | Me | Bn(HOH₂C)CH | H | H | H |
| 266. | H | HN | S | Ph | Bn | Bn(HOH₂C)CH | H | H | H |
| 267. | H | HN | S | 4-OH-Ph | H | Bn(HOH₂C)CH | H | H | H |
| 268. | H | HN | S | 4-OH-Ph | Me | Bn(HOH₂C)CH | H | H | H |

The invention will be better understood in the light of the following Examples.

### EXAMPLE 1

### Preparation of N-benzyl-N'-[2-oxo-2-phenylethyl]-(2R,3R)-2,3-di-O-isopropylidenetartramic Acid Methyl Ester [compound IV wherein R₁ = Ph, R₂ = H, R₃ = PhCH₂, R₄=H, R₅=H, R₆ = COOMe, Z = O, Y = O, R₇-R₈ = CH₂-CH₂]

To a solution of II (wherein R₁ = Ph, R₂ = H, R₃ = PhCH₂) (1.2 g, 5.33 mmol) in anhydrous CH₂Cl₂ (10 ml) (CH₂Cl₂ was filtered through a short pad of anhydrous Na₂CO₃ just before being used) were added, under a nitrogen atmosphere, III (wherein R₄=H, R₅=H, R₆ = COOMe, Z = O, Y = O, R₇-R₈ = CH₂-CH₂) (1.088 g, 5.33 mmol), PyBrOP (2.49 g, 5.33 mmol), and DIPEA (2.73 mL, 15.99 mmol). The mixture was stirred at room temperature for 2 h, and then the solvent was removed to give an oil that was dissolved in EtOAc. This solution was washed with aqueous 5% KHSO₄, 5% NaHCO₃, and brine and dried over Na₂SO₄. After evaporation of the solvent, the crude product obtained was purified by chromatography (EtOAc-petroleum ether, 1:3, Rf 0.32), yielding IV (wherein R₁ = Ph, R₂ = H, R₃ = PhCH₂, R₄=H, R₅=H, R₆ = COOMe, Z = O, Y = O, R₇-R₈ = CH₂-CH₂) (1.645 g, 75%) as a colorless oil:
¹H NMR (CDCl₃): 7.90-7.85 (m, 2H), 7.61-7.22 (m, 8H), 5.39 (d, J = 5.1Hz, 1H), 5.11 (d, J = 5.1Hz, 1 H), 4.88-4.10 (m, 4H), 3.80 (s, 3 H), 1.49 (s, 3 H), 1.31 (s, 3 H).

### EXAMPLE 2

### Preparation of Methyl (1R,5S,7R)-3-Benzyl-2-oxo-5-phenyl-6,8-dioxa-3-azabicyclo[3.2.1]octane-7-exo-carboxylate [compound I wherein R₁ = Ph, R₂ = H, R₃ = PhCH₂, R₄ = H, R₅ = H, R₆ = COOMe, X = O, Z = O, Y = O]

A solution of IV (prepared according the example 1, wherein R₁ = Ph, R₂ = H, R₃ = PhCH₂, R₄=H, R₅=H, R₆ = COOMe, Z = O, Y = O, R₇-R₈ = CH₂-CH₂) (1.645 g, 4.00 mmol) in toluene (40 mL) was quickly added to a refluxing suspension of H₂SO₄/SiO₂ (30% w/w, 700 mg) in toluene (60 mL). The mixture was allowed to react for 15 min, and afterward, one-third of the solvent was distilled off. The hot reaction mixture was filtered through a short layer of NaHCO₃, and after evaporation of the solvent, the crude product was purified by chromatography as above affording pure I (wherein R₁ = Ph, R₂ = H, R₃ = PhCH₂, R₄ = H, R₅ = H, R₆ = COOMe, X = O, Z = O, Y = O) (1.200 g, 85%): mp 112 -114 °C;
[α]²⁵_{D} - 64.3 (*c* 0.8, CDCl₃);
¹H NMR (CDCI3) 7.62-7.59 (m, 2H), 7.41-7.24 (m, 8H), 5.16 (s, 1H), 4.92 (s, 1H), 4.61 (AB system, *J* = 11.0 Hz, 2H), 3.74 (s, 3 H), 3.46 (AB system, *J* = 25.2 Hz, 2H).
¹³C NMR (CDCl₃); 169.0 (s), 165.4(s), 137.8 (s), 135.0 (s), 129.5 (d), 128.8 (d), 128.3 (d), 127.9, 127.8 (d), 125.4 (d), 107.7 (s), 79.1 (d), 78.3 (d), 55.5 (t), 52.6 (q), 48.6 (t)
IR (CDCl₃): 1762, 1678 cm⁻¹
MS (m/z, %): 353 (M⁺, 3), 147 (5), 120(36), 306 (13), 105 (80), 91 (100).

### EXAMPLE 3

### Preparation of N-(p-Methoxybenzyl)-N'-[2-oxo-2-phenylethyl]-(2R,3R)-2,3-di-O-isopropylidenetartramic Acid Methyl Ester [compound IV, wherein R₁ = Ph, R₂ = H, R₃ = 4-MeO-C₆H₄CH₂, R₄=H, R₅=H, R₆ = COOMe, Z = O, Y = O, R₇-R₈ = CH₂-CH₂]

A solution of II (wherein R₁ = Ph, R₂ = H, R₃ = 4-MeO-C₆H₄CH₂) (0.5 g, 2.09 mmol) in anhydrous CH₂Cl₂ (5 ml), III (wherein R₄=H, R₅=H, R₆ = COOMe, Z = O, Y = O, R₇-R₈ = CH₂-CH₂) (0.427 g, 2.09 mmol), PyBrOP (0.976 g, 2.09 mmol), and DIPEA (1.07 mL, 6.27 mmol) was treated as in the example 1. The crude product obtained was purified by chromatography (EtOAc-petroleum ether, 1:3, Rf 0.32), yielding IV (wherein R₁ = Ph, R₂ = H, R₃ = 4-MeO-C₆H₄CH₂, R₄=H, R₅=H, R₆ = COOMe, Z = O, Y = O, R₇-R₈ = CH₂-CH₂) (0.370 g, 40%) as a colorless oil:
¹H NMR (CDCl₃): 7.90-7.85 (m, 2H), 7.61-7.43 (m, 3H), 7.21-7.15 (m, 2H), 6.90-6.82 (m, 2H), 5.39 (d, *J* = 5.1Hz, 1H), 5.13 (d, *J* = 5.1Hz, 1 H), 4.75 (m, 2H), 4.11 (m, 2H), 3.82 (s, 3 H), 3.79 (s, 3 H), 1.52 (s, 3 H), 1.36 (s, 3 H).

### EXAMPLE 4

### Preparation of Methyl (1R,5S,7R)-3-(p-Methoxybenzyl)-2-oxo-5-phenyl-6,8-dioxa-3-azabicyclo[3.2.1]octane-7-exo-carboxylate [compound I wherein R₁ = Ph, R₂ = H, R₃ = 4-MeO-C₆H₄CH₂, R₄ = H, R₅ = H, R₆ = COOMe, X = O, Z = O, Y = O]

A solution of IV (wherein R₁ = Ph, R₂ = H, R₃ = 4-MeO-C₆H₄CH₂, R₄=H, R₅=H, R₆ = COOMe, Z = O, Y = O, R₇-R₈ = CH₂-CH₂) (0.370 g, 0.84 mmol) in toluene (10 mL) was treated as reported in example 2. The crude product was purified by chromatography as above affording pure I (wherein R₁ = Ph, R₂ = H, R₃ = 4-MeO-C₆H₄CH₂, R₄ = H, R₅ = H, R₆ = COOMe, X = O, Z = O, Y = O) (0.177 g, 55 %): mp 134 - 136 °C;
[α]²⁵_{D} - 62.3 (*c* 0.6, CDCl₃);
¹H NMR (CDCI3) 7.62-7.59 (m, 2H), 7.41-7.24 (m, 5H), 7.11-6.91 (m, 2H), 5.14 (s, 1H), 4.89 (s, 1H), 4.24 (AB system, *J* = 11.0 Hz, 2H), 3.78 (s, 3 H), 3.74 (s, 3 H), 3.56 (AB system, *J* = 23.4 Hz, 2H).
¹³C NMR (CDCl₃); 169.4 (s), 165.3(s), 159.8 (s), 137.8 (s), 135.0 (s), 129.5 (d), 128.1 (d), 127.1 (d), 126.4 (d), 119.2 (d), 107.1 (s), 79.8 (d), 78.0 (d), 58.5 (t), 55.1 (q), 52.6 (q), 48.1 (t).
IR (CDCl₃): 1768, 1682 cm⁻¹
MS (m/z, %): 383 (M⁺, 5), 121 (100).

### EXAMPLE 5

Preparation of (1*R*,5*S*,7*R*)-3-Benzyl-2-oxo-5-(4-hydroxyphenyl)-6,8-dioxa-3-azabicyclo[3.2.1]octane-7-*exo*-carboxylic Acid [compound I wherein R₁ = 4-OH-C₆H₄, R₂ = H, R₃ = PhCH₂, R₄ = H, R₅ = H, R₆ = COOH, X = O, Z = O, Y = O] Wang resin (1 g, 200-400 mesh, substitution 0.64 mmol/g) was suspended in CH₂Cl₂ (10 mL) and magnetically stirred for 15 min. After filtration, a solution of Ph₃P (1.024g, 3.904 mmol) and 4'-hydroxy-2-chloroacetophenone (compound V wherein Hal = Cl, R₁ = 4-OH-C₆H₄, R₂ = H), (0.568 g, 3.33 mmol) in a mixture of CH₂Cl₂ (10 mL) and Et₂O (4 mL) was added to the expanded resin. After 5 min, DEAD (607 mL, 3.904 mmol) was added drop-wise and the resulting suspension stirred at room temperature. After 24 h the suspension was filtered and the resin washed with DMF (3 x 10 mL), CH₂Cl₂ (3 x 10 mL), MeOH (3 x 10 mL) and again DMF (3 x 10 mL). Then, the resin (1.00 g), suspended in CH₂Cl₂ (1 mL), was treated with benzylamine (compound VI wherein R₃ = PhCH₂) (10 mL) and left under stirring at room temperature for 12 h. After filtration, the resin II (R₁ = Wang-4-OH-C₆H₄, R₂ = H, R₃ = PhCH₂) obtained was washed as above with DMF, CH₂Cl₂, MeOH and again DMF. Resin II (R₁ = Wang-4-OH-C₆H₄, R₂ = H, R₃ = PhCH₂) was then coupled with III [wherein R₄=H, R₅=H, R₆ = COOMe, Z = O, Y = O, R₇-R₈ = CH₂-CH₂] as follows: compound III (261 mg, 1.28 mmol) and PyBroP (597 mg, 1.28 mmol) were added to resin II (500 mg) suspended in DMF (10 mL), then DIPEA (438 µL, 1.28 mmol) was added slowly at room temperature and the resulting suspension stirred for 12 h. After the usual work-up, resin IV [R₁ = Wang-4-OH-C₆H₄, R₂ = H, R₃ = PhCH₂, R₄=R₅=H, R₆ = COOMe, Z = O, Y = O, R₇-R₈ = CH₂-CH₂] was obtained. The cyclization step was performed on 250 mg of resin IV as follows: resin IV (250 mg) and *p*-TsOH (6 mg) were suspended in toluene and the mixture refluxed for 15 min. Then part of the solvent (25 mL) was distilled off and the residual suspension filtered. The solution was concentrated obtaining, as a yellow oil, compound I [wherein R₁ = 4-OH-C₆H₄, R₂ = H, R₃ = PhCH₂, R₄ = H, R₅ = H, R₆ = COOH, X = O, Z = O, Y = O] (33 mg). with complete cleavage from the resin.
¹H NMR (CDCl₃) δ: 7.78 (d, *J* = 8.8 Hz, 2 H), 7.60 (d, *J* = 7.2 Hz, 2 H), 7.40-7.00 (m, 3 H), 6.80 (d, *J* = 8.8 Hz, 2 H), 5.13 (s, 1 H), 4.86 (s, 1H), 4.58 (AB system, *J* = 15.0 Hz, 2 H), 3.57 (d, *J* = 11.8 Hz, 1 H), 3.38 (d, *J* = 11.8 Hz, 1 H).

### EXAMPLE 6

### Preparation of N-(4-methylphenyl)-N'-[2-oxo-2-phenylethyl]-(2R,3R)-2,3-di-O-isopropylidenetartramic Acid Methyl Ester [compound IV wherein R₁ = Ph, R₂ = H, R₃ = 4-Me-C₆H₄, R₄=H, R₅= H, R₆ = COOMe, Z = O, Y = O, R₇-R₈ = CH₂-CH₂]

To a solution of III (wherein R₄=H, R₅=H, R₆ = COOMe, Z = O, Y = O, R₇-R₈ = CH₂-CH₂) (366 mg, 1.8 mmol) in methylene chloride (1.8 ml) and PyBrop (839 mg, 1.8 mmol) was added II (wherein R₁ = Ph, R₂ = H, R₃ = 4-Me-C₆H₄) (406mg, 1.8 mmol) and DIPEA (0.765 mL, 3.6 mmol). The mixture was treated as reported in Example 1. The crude product was purified by column chromatography on silica gel (AcOEt- Petroleum Ether. 1:2, R_{f} = 0.37) to give IV (R₁ = Ph, R₂ = H, R₃ = 4-Me-C₆H₄, R₄=R₅= H, R₆ = COOMe, Z = O, Y = O, R₇-R₈ = CH₂-CH₂) as yellow oil (440 mg, 62%).
¹H NMR δ 8.00-7.90 (m, 2H), 7.62-7.39 (m, 4H), 7.36-7.12 (m, 3H), 5.26 (*J*=17.2 Hz part A of AB system, 1H) 4.96 (*J*=17.2 Hz part B of AB system, 1H), 5.07 (*J*=6.6 Hz part A of AB system, 1H) 4.66 (*J*=6.6 Hz part B of AB system, 1H),3.74 (s, 3H), 2.36 (s, 3H), 1.53 (s, 3H), 1.36 (s, 3H). MS (m/z, %): 411 (M⁺, 4), 352 (6), 306 (13), 120(100).

### EXAMPLE 7

### Preparation of Methyl (1R,5S,7R)-3-(4'-methylphenyl)-2-oxo-5-phenyl-6,8-dioxa-3-azabicyclo[3.2.1]octane-7-exo-carboxylate [compound I wherein R₁ = Ph, R₂ = H, R₃ = 4-Me-C₆H₄, R₄ = H, R₅ = H, R₆ = COOMe, X = O, Z = O, Y = O]

A solution of IV (prepared in the example 6, wherein R₁ = Ph, R₂ = H, R₃ = 4-Me-C₆H₄, R₄=H, R₅= H, R₆ = COOMe, Z = O, Y = O, R₇-R₈ = CH₂-CH₂) (310 mg, 0.75 mmol) in toluene (32 ml) was quickly added to a refluxing solution of H₂SO₄/SiO₂ (175 mg) in toluene (16 ml). The mixture was treated as reported in Example 2. The product I [wherein R₁ = Ph, R₂ = H, R₃ = 4-Me-C₆H₄, R₄ = H, R₅ = H, R₆ = COOMe, X = O, Z = O, Y = O] was obtained in pure form (260 mg, 97%). ¹H NMR δ: 7.78-7.66 (m, 2H), 7.48-7.36 (m, 4H), 7.30-7.10 (m, 3H), 5.23 (s, 1H), 5.02 (s, 1H), 4.02 (*J*=12 Hz part A of AB system, 1H) 3.90 (*J*=12 Hz part B of AB system, 1H), 3.73 (s, 3H), 2.35 (s, 3H).¹³C NMR δ: 168.9(s), 165.1(s), 137.4 (s), 136.8 (s), 135.1(s), 129.9 (d), 129.6 (d), 128.4 (d), 125.4 (d), 125.3(d), 107.6 (s),79.4 (d), 78.4 (d), 59.2 (t), 52.7 (q), 20.9 (q). MS (m/z, %): 353 (M⁺,4), 294 (2), 119 (100).

### EXAMPLE 8

### Preparation of N-[(1S)-(1-carbomethoxy-2-phenylethyl)]-N'-[2-oxo-2-phenylethyl]-(2R,3R)-2,3-di-O-isopropylidenetartramic Acid Methyl Ester [compound IV wherein R₁ = Ph, R₂ = H, R₃ = CH(COOMe)CH₂Ph, R₄=H, R₅= H, R₆ = COOMe, Z = O, Y = O, R₇-R₈ = CH₂-CH₂].

To a solution of III (wherein R₄=H, R₅=H, R₆ = COOMe, Z = O, Y = O, R₇-R₈ = CH₂-CH₂) (118 mg, 0.58 mmol) in CH₂Cl₂ (0.5 mL), and PyBrOP (270 mg, 0.58 mmol) was added II (wherein R₁ = Ph, R₂ = H, R₃ = CH(COOMe)CH₂Ph) (120 mg, 0.4 mmol) and DIPEA (0.255 mL, 1.2 mmol). The mixture was treated as reported in Example 1. The crude product was purified by column chromatography on silica gel (CH₂Cl₂ - MeOH (40:1) to afford IV (wherein R₁ = Ph, R₂ = H, R₃ = CH(COOMe)CH₂Ph, R₄=H, R₅ = H, R₆ = COOMe, Z = O, Y = O, R₇-R₈ = CH₂-CH₂) (160mg, 82%).

The ¹H and ¹³C NMR spectrums show two set of signals in 2:1 ratio. ¹H NMR (CDCl₃) δ: 8.04-7.90 (m, 2H), 7.70-7.42 (m,.4H), 7.38-7.20 (m, 4H), 5.48-4.74 (m, 5H), 3.76 and 3.75 (s,3H), 3.59 (s,3H), 3.38-3.30 (m, 2H), 1.56 and 1.46, 1.33, 1.28 (s, 6H). ¹³C NMR (CDCl₃) δ:193.6, 192.3, 170.7, 170.6, 169.9, 169.4, 168.5, 136.6, 135.9, 135.0, 134.5, 133.7, 129.1, 129.0, 128.7, 128.5, 128.4, 128.3, 127.8, 127.6, 126.8, 126.6, 113.2, 77.2, 76.9, 75.4, 60.3, 59.3, 52.5, 52.3, 51.7, 49.2, 36.4, 35.6, 26.5, 26.3, 26.2, 25.9. MS m/z (%): 483 (M+, 2), 424 (4), 378 (7), 320 (16), 206 (34), 192 (50), 162 (63), 105 (100)

### EXAMPLE 9

### Preparation of Methyl (1R,5S,7R)-3-[(1S)-1-carbomethoxy-2-phenylethyl]-2-oxo-5-phenyl-6,8-dioxa-3-azabicyclo[3.2.1]octane-7-exo-carboxylate [compound I wherein R₁ = Ph, R₂ = H, R₃ = CH(COOMe)CH₂Ph, R₄ = H, R₅ = H, R₆ = COOMe, X = O, Z = O, Y = O]

A solution of IV (prepared according the example 8, wherein R₁ = Ph, R₂ = H, R₃ = CH(COOMe)CH₂Ph, R₄=H, R₅= H, R₆ = COOMe, Z = O, Y = O, R₇-R₈ = CH₂-CH₂) (150 mg, 0.30 mmol) in toluene (5 ml) was quickly added to a refluxing solution of H₂SO₄/SiO₂ (60 mg) in toluene (33 ml) The mixture was treated as reported in Example 2. The crude product was purified by flash chromatography (AcOEt-Petroleum Ether 1:1, R*_{f}* = 0.41) to afford I (wherein R₁ = Ph, R₂ = H, R₃ = CH(COOMe)CH₂Ph, R₄ = H, R₅ = H, R₆ = COOMe, X = O, Z = O, Y = O) as 2:1 mixture of epimers (82 mg, 65%).
¹H NMR (CDCl₃) major epimer: δ 7.60 (m, 2 H), 7.90-7.30 (m, 8 H), 5.11 (dd, *J* = 5.6, 10.8 Hz, 1 H), 4.99 (s, 1 H), 4.84 (s 1 H), 3.78 (s, 3 H), 3.72 (s, 3 H), 3.75-3.34 (m, 3 H), 3.08 (m, 1 H).
MS m/z (%):425 (M⁺, 2), 366 (19), 306 (7), 192 (32), 105 (100), 91 (88), 77 (62).

### EXAMPLE 10

### Preparation of N-Boc N-(4-methyoxybenzyl)-N'-[2-oxo-2-phenylethyl]-threoninamide IV (wherein R₁ = Ph, R₂ = H, R₃ = p-CH₃O-C₆H₄CH₂, R₄ = H, R₅ = H, R₆= Me, R₇ = Boc, R₈ = H, Z = N, Y = O).

To a solution of III (R₄ = H, R₅ = H, R₆ = Me, R₇ = Boc, R₈ = H, Z = N, Y = O) in CH₂Cl₂ (5 mL) and PyBrOP (531 mg, 1.14 mmol) was added II (wherein R₁ = Ph, R₂ = H, R₃ = *p*-CH₃O-C₆H₄CH₂) (333 mg, 1.14 mmol) and DIPEA (0.585 mL, 3.42 mmol). The mixture The mixture was treated as reported in Example 1. The crude product was purified by column chromatography on silica gel (EtOAc-petrolrum ether, 1:1.5, R*_{f}* = 0.23) to afford IV (wherein R₁ = Ph, R₂ = H, R₃ = *p*-CH₃O-C₆H₄CH₂, R₄ = H, R₅ = H, R₆ = Me, R₇ = Boc, R₈ = H, Z = N, Y = O) (232 mg, 44%) as an oil.
¹H NMR (CDCl₃) (1:1 mixture of rotamers) δ 7.85 (d, *J* = 7.3 Hz, 2 H), 7.55 (m, 1 H), 7.42 (m, 2 H), 7.11(m, 2 H), 6.82 (m, 2 H), 5.50 (m, 1 H), 5.29 (d, *J* = 14.3 Hz, 1 H), 5.00-4.20 (m, 5 H), 4.00 (m, 1 H), 3.78 (s, 3 H), 3.76 (s, 3 H), 1.38 (s, 9 H), 1.31 (s, 9 H), 1.19 (d, *J* = 6.2 Hz, 3 H), 1.07 (d, *J* = 6.2 Hz, 3 H).

### EXAMPLE 11

### Preparation of (1R,5S,7R)-3-(4-methoxybenzyl)-2-oxo-5-phenyl-7-exo-methyl-6 oxa-3,8-diazabicyclo[3.2.1]octane [compound I wherein R₁ = Ph, R₂ = H R₃ = p-CH₃O-C₆H₄CH₂, R₄ = H, R₅ = H, R₆ = Me, X = O, Z = N, Y = O]

A solution of IV (wherein R₁ = Ph, R₂ = H, R₃ = *p*-CH₃O-C₆H₄CH₂, R₄ = H, R₅ = H, R₆ = Me, R₇ = Boc, R₈ = H, Z = N, Y = O) (78.3 mg, 0.172 mmol) and *p*-TsOH (36 mg, 0.189 mmol) in benzene (10 ml) is refluxed for 30 min, then 8 ml of solvent were distilled off. The resulting solution was concentrated obtaining compound I (I wherein R₁ = Ph, R₂ = H R₃ = *p*-CH₃O-C₆H₄CH₂, R₄ = H, R₅ = H, R₆ = Me, X = O, Z = N, Y = O) as p-TsOH salt (60 mg, 76%). This was treated with 0.1 M aqueous solution of KOH end the free amine extracted with CHCl₃ to give, after concentration, compound I (I wherein R₁ = Ph, R₂ = H R₃ = *p*-CH₃O-C₆H₄CH₂, R₄ = H, R₅ = H, R₆ = Me, X = O, Z = N, Y = O) as a colorless oil (41 mg, 70%).
¹H NMR (CDCl₃) δ 7.70 (m, 2 H), 7.52-7.20 (m, 5 H), 6.83 (m, 2 H), 5.07 (s, 1 H), 4.79 (d, *J* = 14.1 Hz, 1 H), 4.55 (d, *J* = 14.1 Hz, 1 H), 3.78 (s, 3 H), 3.78 (m, 2 H), 2.84 (q, *J* = 7.4 Hz, 1 H), 1.60 (d, *J* = 7.4 Hz, 3 H).

### EXAMPLE 12

### Preparation of (1S,5S,7S)-3-benzyl-5-phenyl-7-exo-hydroxymethyl-6,8-dioxa-3-azabicyclo[3.2.1]octane [compound I wherein R₁ = Ph, R₂ = H, R₃ = PhCH₂, R₄ = H, R₅ = H, R₆ = CH₂OH, X = H , Z = O, Y = O]

To a suspension of LiAlH₄ (50 mg, mmol) in anhydrous THF (10 mL) was added dropwise at 0 °C and under nitrogen atmosphere a solution of compound I, [prepared according the example 2, wherein R₁ = Ph, R₂ = H, R₃ = PhCH₂, R₄ = H, R₅ = H, R₆ = COOMe, X = O, Z = O, Y = O] (22 mg, 0.568 mmol) in dry THF (12 ml). The mixture was refluxed for 2h, and then, after cooling to 0 °C, diethyl ether (2 mL) were added. The mixture was filtered through a short layer of anhydrous Na₂SO₄, and the residue was suspended in 1 M KOH solution (30 mL), saturated with NaCI, and extracted with Et₂O and EtOAc. The organic phases were combined, dried over anhydrous Na₂SO₄ and concentrated to give compound I (wherein R₁ = Ph, R₂ = H, R₃ = PhCH₂, R₄ = H, R₅ = H, R₆ = CH₂OH, X = H , Z = O, Y = O) as a colorless oil (35 mg, 0.112 mmol, 79 %).
¹H NMR (CDCl₃) δ 7.53-7.30 (m, 2 H), 7.29-7.23 (m, 8 H), 4.66-4.34 (m, 2 H), 3.34-3.46 (m, 4 H), 3.06-2.43 (m. 4 H), 1.82 (br s, 1 H).

### EXAMPLE 13

### Preparation of Methyl (1R,5S,7R)-3-[(1S)-1-carbomethoxy-2-phenylethyl]-2-oxo-5-phenyl-6,8-dioxa-3-azabicyclo[3.2.1]octane-7-exo-carboxylate [compound I wherein R₁ = Ph, R₂ = H, R₃ = CH(COOMe)CH₂Ph, R₄ = H, R₅ = H, R₆ = COOMe, X = O, Z = O, Y = O]

Fmoc-(S)-phenylalanine-O-Wang resin (2 g, 200-400 mesh, substitution 1 mmol/g) was treated with piperidine (30%) in DMF (10 mL) under stirring, for 15 min, to obtain compound VI [wherein R₃ =CH(COO-Wang resin)CH₂Ph]. After filtration, the resin suspended in DMF (10 mL), was treated with 2-bromo-acetophenone (compound V wherein Hal = Br, R₁ = Ph, R₂ = H), (1.09 g, 6.0 mmol) and DIPEA (340 µL, 2 mmol) and left under stirring at room temperature for 48 h. The resin II [R₁ =Ph, R₂ = H R₃ =CH(COO-Wang resin)CH₂Ph] obtained was washed as reported in example 5 with DMF, CH₂Cl₂, MeOH and again DMF. Resin II [R₁ = Ph, R₂ = H R₃ =CH(COO-Wang resin)CH₂Ph] was then coupled with III [wherein R₄=H, R₅=H, R₆ = COOMe, Z = O, Y = O, R₇-R₈ = CH₂-CH₂] as follows: compound III (816 mg, 4 mmol) and PyBroP (1.86 g, 4 mmol) were added to resin II (1.00 g) suspended in DMF (10 mL), then DIPEA (680 µL, 4 mmol) was added slowly at room temperature and the resulting suspension stirred for 12 h. After the usual work-up, resin IV [R₁ = Ph, R₂ = H, R₃ =CH(COO-Wang resin)CH₂Ph, R₄=H, R₅=H, R₆ = COOMe, Z = O, Y = O, R₇-R₈ = CH₂-CH₂] was obtained. The cyclization step was performed on 1 g of resin IV as follows: resin IV (1 g) and *p*-TsOH (95 mg) were suspended in toluene and the mixture refluxed for 15 min. Then part of the solvent (50 mL) was distilled off and the residual suspension filtered. The solution was concentrated obtaining, a solid residue (170 mg) contaning compound I [wherein R₁ = Ph, R₂ = H, R₃ = CH(COOH)CH₂Ph, R₄ = H, R₅ = H, R₆ = COOH, X = O, Z = O, Y = O].
Crude compound I [wherein R₁ = Ph, R₂ = H, R₃ = CH(COOH)CH₂Ph, R₄ = H, R₅ = H, R₆ = COOH, X = O, Z = O, Y = O] treated with solution of diazomethane in ether gave compound I [wherein R₁ = Ph, R₂ = H, R₃ = CH(COOMe)CH₂Ph, R₄ = H, R₅ = H, R₆ = COOMe, X = O, Z = O, Y = O] identical with the product (major epimer) as described in example 8.

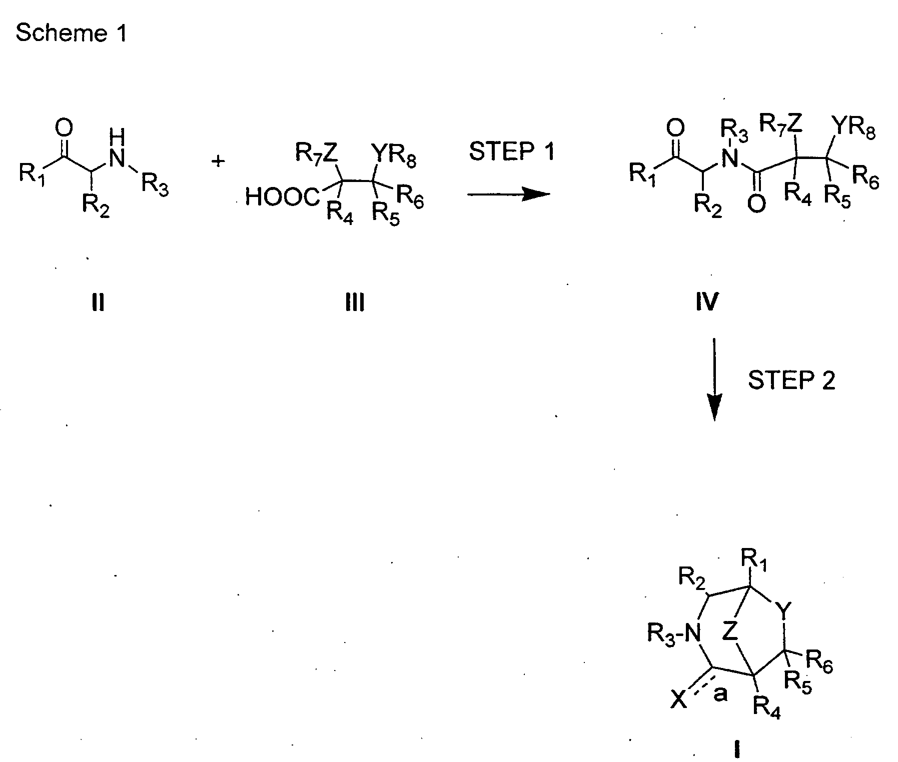

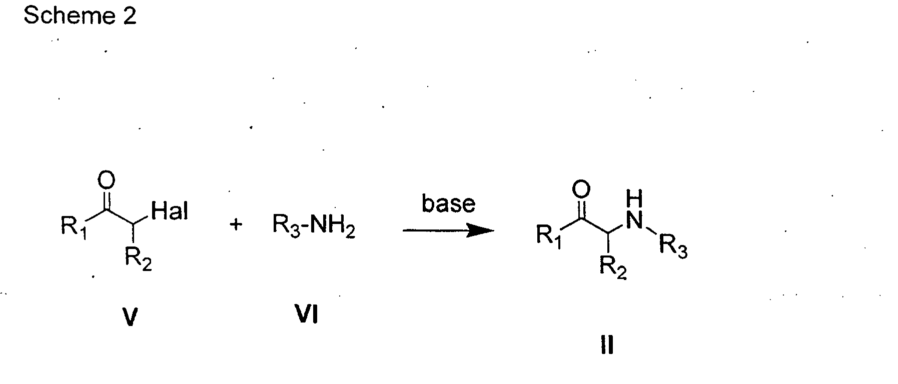

## Claims

1. Heterobicycle derivatives of general formula (I) 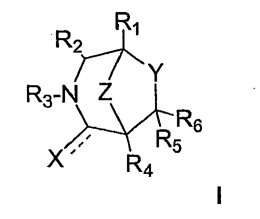 wherein:
R₁, is chosen in the group consisting of C₁₋₈alkyl, C₂₋₈alkenyl, C₂₋₈alkinyl, cycloalkyl, aryl, heterocycle, arylC₁₋₈alkyl; heterocycleC₁₋₈alkyl; RR'N-C₁₋₈alkyl, RR'N-aryl, RO-aryl, R(O)C-aryl, RO(O)C-aryl, RR'N(O)C-aryl, (P)-W-NR-aryl, (P)-W-O-aryl, (P)-W-C(O)O-aryl, (P)-W-O(O)C-aryl, (P)-W-C(O)RN-aryl, (P)-W-NR(O)C-aryl;
R₂, is chosen in the group consisting of H, C₁₋₈alkyl, C₂₋₈alkenyl, C₂₋₈alkinyl, cycloalkyl, aryl, arylC₁₋₈alkyl; heterocycleC₁₋₈alkyl; aminoC₁₋₈alkyl, aminoaryl, C₁₋₈ alkyloxyaryl, hydroxyaryl, carboxyaryl, carboalkyloxyaryl, alkylcarbamoylaryl,-(side chain), -(side chain)-W-(P) or
R₁ and R₂ taken together are a C₁₋₄alkyl, C₂₋₄alkenyl, cycloalkyl, benzofused cycloalkyl, to form a bridge of 3, 4, 5, 6 terms;
R₃, is chosen in the group consisting H, C₁₋₈alkyl, C₂₋₈alkenyl, C₂₋₈alkinyl, cycloalkyl, aryl, arylC₁₋₈alkyl; heterocycleC₁₋₈alkyl; RR'NC₁₋₈alkyl, RR'Naryl, RO-C₁₋₈ alkyl, RO(O)C-C₁₋₈alkyl, R(O)C-C₁₋₈alkyl, RC(O)O-C₁₋₈alkyl, RC(O)N(R)C₁₋₈alkyl RO-aryl, RO(O)C-aryl, R(O)C-aryl RC(O)O-aryl, RC(O)N(R)aryl, -CH(amino acid side- chain)CO₂R, -CH(amino acid side-chain)C(O)NR, -CH(amino acid side-chain)-C(O)O-W-(P), -CH(amino acid side-chain)-C(O)N(R)-W-(P), CH(CO₂R)-amino acid side-chain-W-(P), CH(CONRR')-amino acid side-chain-W-(P), protecting group;
R₄ and R₅, same or different, are chosen in the group consisting H, C₁₋₈alkyl, C₂₋₈ alkenyl, C₂₋₈alkinyl, cycloalkyl, aryl, heterocycle, arylC₁₋₈alkyl; heterocycleC₁₋₈alkyl;
R₆ is chosen in the group consisting, H, C₁₋₈alkyl, C₂₋₈alkenyl, C₂₋₈alkinyl, cycloalkyl, aryl, arylC₁₋₈alkyl, heterocycle, heterocycleC₁₋₈alkyl; -C(O)R, -C(O)OR, -C(O)NRR', CH₂OR, CH₂NRR', -C(O)NH-CH(amino acid side-chain)C(O)OR, - C(O)O-W-(P), -C(O)N(R)-W-(P), -CH₂O-W-(P), -CH₂N(R)-W-(P);
R and R', same or different, are chosen in the group consisting of: H, C₁₋₈alkyl, C₂₋₈alkenyl, C₂₋₈alkinyl, cycloalkyl, aryl, heterocycle, arylC₁₋₈alkyl; heterocycleC₁₋₈ alkyl; a protecting group, -C(O)CH-(amino acid side-chain)-NHR, -NH-CH(amino acid side-chain)COOR, -CH(amino acid side-chain)COOR;
P is resin, both soluble or bound to a solid support;
W is as linker;
X is O, S, when a is a double bond, or X is H and a is single bond,
Y and Z, same or different, are O, S, SO, SO₂, N-R, wherein R is as above defined;
wherein the term cycloalkyl represents: cyclopropane, cyclobutane, cyclopentane, cyclohexane, cycloheptane, cyclooctane, norbornane, canphane, adamantine; the term aryl specifies phenyl, biphenyl and naphtyl groups substituted with one or two moieties chosen from the groups consisting of halogen, cyano, nitro, C₁₋₆ alkyl; the term heterocycle represents pyridine, imidazole, pyrrole, indole, triazoles, pyrrolidine, pyperidine and the term protecting group refers to known groups capable of preventing reaction or bonding of oxygen, nitrogen, carboxylic acids, thiols, alcohols, amines and the like;
the above said alkyl-, alkenyl-, alkinyl-, cycloalkyl-, aryl- and heterocycle-groups, being possibly substituted with the proviso that:
when: Z = Y = X = O, R₂= R₄ = R₅ = H, R₃ = CH₃ and R₆ = Ph then R₁₌is not Ph;
and
when X = R₂ = H and Y = Z = O then at least one R₄, R₅ or R₆ is not H.

2. Heterobicycle derivatives according to Claim 1 wherein:
the resin P is a polymeric material soluble in the solvents commonly used in organic synthesis or bound to a solid support;
the solid support is a solid material (at room temperature) to which starting resin materials (reactive groups) may be bound;
W is a molecule capable of binding the resin P to the reagents and the products of formula (I);
Amino acid side-chain means the side chain moieties of the natural occurring L or D amino acids or the non naturally occurring amino acids;
and the other substituents are as definied in Claim 1.

3. Heterobicycle derivatives according to Claim 2 wherein:
the resin is a polymeric material derivatised with a -NH₂ group or an hydroxyl group possibly bound to a solid support materials chosen among polyethylene and polystyrene compounds and related inert polymeric compounds;
the amino acid side-chain is the side chain of a naturally or non naturally occurring amino acid and the other substituents are as defined in Claim 1.

4. Heterobicycle derivatives according to Claim 3 wherein the non naturally occurring amino acids are chosen among. norleucine (Nle), norvaline (NVa), β-alanine, L or D α-phenyl glycine and the like and the other substituents are as described in Claim 1.

5. Heterobicycle derivatives according to Claim 4 represented by the following formulae:
| Comp. | X | Z | Y | R₁ | R₂ | R₃ | R₄ | R₅ | R₆ |
|---|---|---|---|---|---|---|---|---|---|
| 1. | O | O | O | Ph | H | PhCH₂ | H | H | COOH |
| 2. | O | O | O | 4-HO-Ph | H | PhCH₂ | H | H | COOH |
| 3. | O | O | O | 4-O₂N-Ph | H | PhCH₂ | H | H | COOH |
| 4. | O | O | O | 4-H₂N-Ph | H | PhCH₂ | H | H | COOH |
| 5. | O | O | O | 4-MeO(O)C-Ph | H | PhCH₂ | H | H | COOH |
| 6. | O | O | O | 4-Me-Ph | H | PhCH₂ | H | H | COOH |
| 7. | O | O | O | 4-MeO-Ph | H | PhCH₂ | H | H | COOH |
| 8. | O | O | O | 4-Cl-Ph | H | PhCH₂ | H | H | COOH |
| 9. | O | O | O | 4-Br-Ph | H | PhCH₂ | H | H | COOH |
| 10. | O | O | O | 2-HO-Ph | H | PhCH₂ | H | H | COOH |
| 11. | O | O | O | 2-O₂N-Ph | H | PhCH₂ | H | H | COOH |
| 12. | O | O | O | 2-H₂N-Ph | H | PhCH₂ | H | H | COOH |
| 13. | O | O | O | 2-MeO(O)C-Ph | H | PhCH₂ | H | H | COOH |
| 14. | O | O | O | 2-Me-Ph | H | PhCH₂ | H | H | COOH |
| 15. | O | O | O | 2-MeO-Ph | H | PhCH₂ | H | H | COOH |
| 16. | O | O | O | 2-Cl-Ph | H | PhCH₂ | H | H | COOH |
| 17. | O | O | O | 2-Br-Ph | H | PhCH₂ | H | H | COOH |
| 18. | O | O | O | 2-Nafthyl | H | PhCH₂ | H | H | COOH |
| 19. | O | O | O | 2-thienyl | H | PhCH₂ | H | H | COOH |
| 20. | O | O | O | 4-biphenyl | H | PhCH₂ | H | H | COOH |
| 21. | O | O | O | Ph | H | Me | H | H | COOH |
| 22. | O | O | O | Ph | H | CH₃(CH₂)₂ | H | H | COOH |
| 23. | O | O | O | Ph | H | cyclohexyl | H | H | COOH |
| 24. | O | O | O | Ph | H | allyl | H | H | COOH |
| 25. | O | O | O | Ph | H | Ph | H | H | COOH |
| 26. | O | O | O | Ph | H | 4-HO-Ph | H | H | COOH |
| 27. | O | O | O | Ph | H | 4-O₂N-Ph | H | H | COOH |
| 28. | O | O | O | Ph | H | 4-MeO₂C-Ph | H | H | COOH |
| 29. | O | O | O | Ph | H | 4-Me-Ph | H | H | COOH |
| 30. | O | O | O | Ph | H | 4-MeO-Ph | H | H | COOH |
| 31. | O | O | O | Ph | H | 4-Cl-Ph | H | H | COOH |
| 32. | O | O | O | Ph | H | 4-Br-Ph | H | H | COOH |
| 33. | O | O | O | Ph | H | 2-HO-Ph | H | H | COOH |
| 34. | O | O | O | Ph | H | 2-O₂N-Ph | H | H | COOH |
| 35. | O | O | O | Ph | H | 2-MeO₂C-Ph | H | H | COOH |
| 36. | O | O | O | Ph | H | 2-Me-Ph | H | H | COOH |
| 37. | O | O | O | Ph | H | 2-MeO-Ph | H | H | COOH |
| 38. | O | O | O | Ph | H | 2-Cl-Ph | H | H | COOH |
| 39. | O | O | O | Ph | H | 2-Br-Ph | H | H | COOH |
| 40. | O | O | O | Ph | H | 2-Nafthyl | H | H | COOH |
| 41. | O | O | O | Ph | H | 2-thienyl | H | H | COOH |
| 42. | O | O | O | Ph | H | 4-biphenyl | H | H | COOH |
| 43. | O | O | O | Ph | H | 4-MeO₂C-PhCH₂ | H | H | COOH |
| 44. | O | O | O | Ph | H | 4-Me-PhCH₂ | H | H | COOH |
| 45. | O | O | O | Ph | H | 4-MeOPhCH₂ | H | H | COOH |
| 46. | O | O | O | Ph | H | 4-Cl-PhCH₂ | H | H | COOH |
| 47. | O | O | O | Ph | H | 4-Br-PhCH₂ | H | H | COOH |
| 48. | O | O | O | Ph | H | 2-HO-PhCH₂ | H | H | COOH |
| 49. | O | O | O | Ph | H | 2-O₂N-PhCH₂ | H | H | COOH |
| 50. | O | O | O | Ph | H | 2-MeO₂C-PhCH₂ | H | H | COOH |
| 51. | O | O | O | Ph | H | 2-Me-PhCH₂ | H | H | COOH |
| 52. | O | O | O | Ph | H | 2-MeO-PhCH₂ | H | H | COOH |
| 53. | O | O | O | Ph | H | 2-Cl-PhCH₂ | H | H | COOH |
| 54. | O | O | O | Ph | H | 2-Br-PhCH₂ | H | H | COOH |
| 55. | O | O | O | 4-HO-Ph | H | 4-HO-Ph CH₂ | H | H | COOH |
| 56. | O | O | O | 4-HO-Ph | H | 4-O₂N-PhCH₂ | H | H | COOH |
| 57. | O | O | O | 4-HO-Ph | H | 4-MeO₂C-PhCH₂ | H | H | COOH |
| 58. | O | O | O | 4-HO-Ph | H | 4-Me-PhCH₂ | H | H | COOH |
| 59. | O | O | O | 4-HO-Ph | H | 4-MeOPhCH₂ | H | H | COOH |
| 60. | O | O | O | 4-HO-Ph | H | 4-Cl-PhCH₂ | H | H | COOH |
| 61. | O | O | O | 4-HO-Ph | H | 4-Br-PhCH₂ | H | H | COOH |
| 62. | O | O | O | 4-HO-Ph | H | 2-HO-PhCH₂ | H | H | COOH |
| 63. | O | O | O | 4-HO-Ph | H | 2-O₂N-PhCH₂ | H | H | COOH |
| 64. | O | O | O | 4-HO-Ph | H | 2-MeO₂C-PhCH₂ | H | H | COOH |
| 65. | O | O | O | 4-HO-Ph | H | 2-Me-PhCH₂ | H | H | COOH |
| 66. | O | O | O | 4-HO-Ph | H | 2-MeO-PhCH₂ | H | H | COOH |
| 67. | O | O | O | 4-HO-Ph | H | 2-Cl-PhCH₂ | H | H | COOH |
| 68. | O | O | O | 4-HO-Ph | H | 2-Br-PhCH₂ | H | H | COOH |
| 69. | O | O | O | 4-HO-Ph | H | Me | H | H | COOH |
| 70. | O | O | O | 4-HO-Ph | H | CH₃(CH₂)₂ | H | H | COOH |
| 71. | O | O | O | 4-HO-Ph | H | cyclohexyl | H | H | COOH |
| 72. | O | O | O | 4-HO-Ph | H | allyl | H | H | COOH |
| 73. | O | O | O | Ph | H | HO₂C-CH₂ | H | H | COOH |
| 74. | O | O | O | Ph | H | Bn(HO₂C)CH | H | H | COOH |
| 75. | O | O | O | Ph | H | HOCH₂(HO₂C)CH | H | H | COOH |
| 76. | O | O | O | Ph | H | CH₃(HO)CH(HO₂C)CH | H | H | COOH |
| 77. | O | O | O | Ph | H | MeS(CH₂)₂(HO₂C)CH | H | H | COOH |
| 78. | O | O | O | Ph | H | H₂N(CH₂)₃(HO₂C)CH | H | H | COOH |
| 79. | O | O | O | Ph | H | HO₂CCH₂(HO₂C)CH | H | H | COOH |
| 80. | O | O | O | Ph | H | imidazole-CH₂(HO₂C)CH | H | H | COOH |
| 81. | O | O | O | Ph | H | indole-CH₂(HO₂C)CH | H | H | COOH |
| 82. | O | O | O | 4-HO-Ph | H | HO₂C-CH₂ | H | H | COOH |
| 83. | O | O | O | 4-HO-Ph | H | Me(HO₂C)CH | H | H | COOH |
| 84. | O | O | O | 4-HO-Ph | H | (CH₃)₂CH(HO₂C)CH | H | H | COOH |
| 85. | O | O | O | 4-HO-Ph | H | Bn(HO₂C)CH | H | H | COOH |
| 86. | O | O | O | 4-HO-Ph | H | HOCH₂(HO₂C)CH | H | H | COOH |
| 87. | O | O | O | 4-HO-Ph | H | CH₃(HO)CH(HO₂C)CH | H | H | COOH |
| 88. | O | O | O | 4-HO-Ph | H | MeS(CH₂)₂(HO₂C)CH | H | H | COOH |
| 89. | O | O | O | 4-HO-Ph | H | H₂N(CH₂)₃(HO₂C)CH | H | H | COOH |
| 90. | O | O | O | 4-HO-Ph | H | HO₂CCH₂(HO₂C)CH | H | H | COOH |
| 91. | O | O | O | 4-HO-Ph | H | imidazole-CH₂(HO₂C)CH | H | H | COOH |
| 92. | O | O | O | 4-HO-Ph | H | indole-CH₂(HO₂C)CH | H | H | COOH |
| 93. | O | O | O | Ph | Me | PhCH₂ | H | H | COOH |
| 94. | O | O | O | 4-HO-Ph | Me | PhCH₂ | H | H | COOH |
| 95. | O | O | O | Ph | Bn | PhCH₂ | H | H | COOH |
| 96. | O | O | O | 4-HO-Ph | Bn | PhCH₂ | H | H | COOH |
| 97. | O | O | O | Ph | Me | HO₂C-CH₂ | H | H | COOH |
| 98. | O | O | O | 4-HO-Ph | Me | HO₂C-CH₂ | H | H | COOH |
| 99. | O | O | O | Ph | Bn | HO₂C-CH₂ | H | H | COOH |
| 100. | O | O | O | 4-HO-Ph | Bn | HO₂C-CH₂ | H | H | COOH |
| 101. | O | O | O | Ph | Me | Bn(HO₂C)CH | H | H | COOH |
| 102. | O | O | O | 4-HO-Ph | Me | Bn(HO₂C)CH | H | H | COOH |
| 103. | O | HN | O | Ph | H | PhCH₂ | H | H | CH₃ |
| 104. | O | HN | O | Ph | Me | PhCH₂ | H | H | CH₃ |
| 105. | O | HN | O | Ph | Bn | PhCH₂ | H | H | CH₃ |
| 106. | O | HN | O | 4-OH-Ph | H | PhCH₂ | H | H | CH₃ |
| 107. | O | HN | O | 4-OH-Ph | Me | PhCH₂ | H | H | CH₃ |
| 108. | O | HN | O | 4-OH-Ph | Bn | PhCH₂ | H | H | CH₃ |
| 109. | O | HN | O | Ph | H | Ph | H | H | CH₃ |
| 110. | O | HN | O | Ph | Me | Ph | H | H | CH₃ |
| 111. | O | HN | O | Ph | Bn | Ph | H | H | CH₃ |
| 112. | O | HN | O | 4-OH-Ph | H | Ph | H | H | CH₃ |
| 113. | O | HN | O | 4-OH-Ph | Me | Ph | H | H | CH₃ |
| 114. | O | HN | O | 4-OH-Ph | Bn | Ph | H | H | CH₃ |
| 115. | O | HN | O | Ph | H | CH₃-Ph | H | H | CH₃ |
| 116. | O | HN | O | Ph | Me | CH₃-Ph | H | H | CH₃ |
| 117. | O | HN | O | Ph | Bn | CH₃-Ph | H | H | CH₃ |
| 118. | O | HN | O | 4-OH-Ph | H | CH₃-Ph | H | H | CH₃ |
| 119. | O | HN | O | 4-OH-Ph | Me | CH₃-Ph | H | H | CH₃ |
| 120. | O | HN | O | 4-OH-Ph | Bn | CH₃-Ph | H | H | CH₃ |
| 121. | O | HN | O | Ph | H | 4-MeO-PhCH₂ | H | H | CH₃ |
| 122. | O | HN | O | Ph | Me | 4-MeO-PhCH₂ | H | H | CH₃ |
| 123. | O | HN | O | Ph | Bn | 4-MeO-PhCH₂ | H | H | CH₃ |
| 124. | O | HN | O | 4-OH-Ph | H | 4-MeO-PhCH₂ | H | H | CH₃ |
| 125. | O | HN | O | 4-OH-Ph | Me | 4-MeO-PhCH₂ | H | H | CH₃ |
| 126. | O | HN | O | 4-OH-Ph | Bn | 4-MeO-PhCH₂ | H | H | CH₃ |
| 127. | O | HN | O | Ph | H | CH₃-PhCH₂ | H | H | CH₃ |
| 128. | O | HN | O | Ph | Me | CH₃-PhCH₂ | H | H | CH₃ |
| 129. | O | HN | O | Ph | Bn | CH₃-PhCH₂ | H | H | CH₃ |
| 130. | O | HN | O | 4-OH-Ph | H | CH₃-PhCH₂ | H | H | CH₃ |
| 131. | O | HN | O | 4-OH-Ph | Me | CH₃-PhCH₂ | H | H | CH₃ |
| 132. | O | HN | O | 4-OH-Ph | Bn | CH₃-PhCH₂ | H | H | CH₃ |
| 133. | | HN | O | Ph | H | Me | H | H | CH₃ |
| 134. | O | HN | O | Ph | H | CH₃(CH₂)₂ | H | H | CH₃ |
| 135. | O | HN | O | Ph | H | cyclohexyl | H | H | CH₃ |
| 136. | O | HN | O | Ph | H | allyl | H | H | CH₃ |
| 137. | O | HN | O | 4-OH-Ph | H | Me | H | H | CH₃ |
| 138. | O | HN | O | 4-OH-Ph | H | CH₃(CH₂)₂ | H | H | CH₃ |
| 139. | O | HN | O | 4-OH-Ph | H | cyclohexyl | H | H | CH₃ |
| 140. | O | HN | O | 4-OH-Ph | H | allyl | H | H | CH₃ |
| 141. | O | HN | O | Ph | H | HO₂C-CH₂ | H | H | CH₃ |
| 142. | O | HN | O | Ph | Me | HO₂-CH₂ | H | H | CH₃ |
| 143. | O | HN | O | Ph | Bn | HO₂C-CH₂ | H | H | CH₃ |
| 144. | O | HN | O | 4-OH-Ph | H | HO₂C-CH₂ | H | H | CH₃ |
| 145. | O | HN | O | 4-OH-Ph | Me | HO₂C-CH₂ | H | H | CH₃ |
| 146. | O | HN | O | 4-OH-Ph | Bn | HO₂C-CH₂ | H | H | CH₃ |
| 147. | O | HN | O | Ph | H | Bn(HO₂C)CH | H | H | CH₃ |
| 148. | O | HN | O | Ph | Me | Bn(HO₂C)CH | H | H | CH₃ |
| 149. | O | HN | O | Ph | Bn | Bn(HO₂C)CH | H | H | CH₃ |
| 150. | O | HN | O | 4-OH-Ph | H | Bn(HO₂C)CH | H | H | CH₃ |
| 151. | O | HN | O | 4-OH-Ph | Me | Bn(HO₂C)CH | H | H | CH₃ |
| 152. | O | HN | O | 4-OH-Ph | Bn | Bn(HO₂C)CH | H | H | CH₃ |
| 153. | H | O | O | Ph | H | PhCH₂ | H | H | COOH |
| 154. | H | O | O | Ph | Me | PhCH₂ | H | H | COOH |
| 155. | H | O | O | Ph | Bn | PhCH₂ | H | H | COOH |
| 156. | H | O | O | 4-HO-Ph | H | PhCH₂ | H | H | COOH |
| 157. | H | O | O | 4-HO-Ph | Me | PhCH₂ | H | H | COOH |
| 158. | H | O | O | 4-HO-Ph | Bn | PhCH₂ | H | H | COOH |
| 159. | H | O | O | Ph | H | HO₂C-CH₂ | H | H | COOH |
| 160. | H | O | O | Ph | Me | HO₂C-CH₂ | H | H | COOH |
| 161. | H | O | O | Ph | Bn | HO₂C-CH₂ | H | H | COOH |
| 162. | H | O | O | 4-HO-Ph | H | HO₂C-CH₂ | H | H | COOH |
| 163. | H | O | O | 4-HO-Ph | Me | HO₂C-CH₂ | H | H | COOH |
| 164. | H | O | O | 4-HO-Ph | Bn | HO₂C-CH₂ | H | H | COOH |
| 165. | H | O | O | Ph | H | Bn(HO₂C)CH | H | H | COOH |
| 166. | H | O | O | Ph | Me | Bn(HO₂C)CH | H | H | COOH |
| 167. | H | O | O | Ph | Bn | Bn(HO₂C)CH | H | H | COOH |
| 168. | H | O | O | 4-HO-Ph | H | Bn(HO₂C)CH | H | H | COOH |
| 169. | H | O | O | 4-HO-Ph | Me | Bn(HO₂C)CH | H | H | COOH |
| 170. | H | O | O | 4-HO-Ph | Bn | Bn(HO₂C)CH | H | H | COOH |
| 171. | H | HN | O | Ph | H | PhCH₂ | H | H | CH₃ |
| 172. | H | HN | O | Ph | H | 4-MeO-PhCH₂ | H | H | CH₃ |
| 173. | H | HN | O | Ph | Me | PhCH₂ | H | H | CH₃ |
| 174. | H | HN | O | Ph | Bn | PhCH₂ | H | H | CH₃ |
| 175. | H | HN | O | 4-OH-Ph | H | PhCH₂ | H | H | CH₃ |
| 176. | H | HN | O | 4-OH-Ph | Me | PhCH₂ | H | H | CH₃ |
| 177. | H | HN | O | 4-OH-Ph | Bn | PhCH₂ | H | H | CH₃ |
| 178. | H | HN | O | Ph | H | HO₂C-CH₂ | H | H | CH₃ |
| 179. | H | HN | O | Ph | Me | HO₂C-CH₂ | H | H | CH₃ |
| 180. | H | HN | O | Ph | Bn | HO₂C-CH₂ | H | H | CH₃ |
| 181. | H | HN | O | 4-OH-Ph | H | HO₂C-CH₂ | H | H | CH₃ |
| 182. | H | HN | O | 4-OH-Ph | Me | HO₂C-CH₂ | H | H | CH₃ |
| 183. | H | HN | O | 4-OH-Ph | Bn | HO₂C-CH₂ | H | H | CH₃ |
| 184. | H | HN | O | Ph | H | Bn(HO₂C)CH | H | H | CH₃ |
| 185. | H | HN | O | Ph | Me | Bn(HO₂C)CH | H | H | CH₃ |
| 186. | H | HN | O | Ph | Bn | Bn(HO₂C)CH | H | H | CH₃ |
| 187. | H | HN | O | 4-OH-Ph | H | Bn(HO₂C)CH | H | H | CH₃ |
| 188. | H | HN | O | 4-OH-Ph | Me | Bn(HO₂C)CH | H | H | CH₃ |
| 189. | H | HN | 0 | 4-OH-Ph | Bn | Bn(HO₂C)CH | H | H | CH₃ |
| 190. | H | HN | O | Ph | H | Ph | H | H | CH₃ |
| 191. | H | HN | O | Ph | Me | Ph | H | H | CH₃ |
| 192. | H | HN | O | Ph | Bn | Ph | H | H | CH₃ |
| 193. | H | HN | O | 4-OH-Ph | H | Ph | H | H | CH₃ |
| 194. | H | HN | O | 4-OH-Ph | Me | Ph | H | H | CH₃ |
| 195. | H | HN | 0 | 4-OH-Ph | Bn | Ph | H | H | CH₃ |
| 196. | H | HN | O | Ph | H | CH₃-Ph | H | H | CH₃ |
| 197. | H | HN | O | Ph | Me | CH₃-Ph | H | H | CH₃ |
| 198. | H | HN | O | Ph | Bn | CH₃-Ph | H | H | CH₃ |
| 199. | H | HN | O | 4-OH-Ph | H | CH₃-Ph | H | H | CH₃ |
| 200. | H | HN | O | 4-OH-Ph | Me | CH₃-Ph | H | H | CH₃ |
| 201. | H | HN | O | 4-OH-Ph | Bn | CH₃-Ph | H | H | CH₃ |
| 202. | H | HN | O | Ph | H | 4-MeO-PhCH₂ | H | H | CH₃ |
| 203. | H | HN | O | Ph | Me | 4-MeO-PhCH₂ | H | H | CH₃ |
| 204. | H | HN | O | Ph | Bn | 4-MeO-PhCH₂ | H | H | CH₃ |
| 205. | H | HN | O | 4-OH-Ph | H | 4-MeO-PhCH₂ | H | H | CH₃ |
| 206. | H | HN | O | 4-OH-Ph | Me | 4-MeO-PhCH₂ | H | H | CH₃ |
| 207. | H | HN | O | 4-OH-Ph | Bn | 4-MeO-PhCH₂ | H | H | CH₃ |
| 208. | H | HN | O | Ph | H | CH₃-PhCH₂ | H | H | CH₃ |
| 209. | H | HN | O | Ph | Me | CH₃-PhCH₂ | H | H | CH₃ |
| 210. | H | HN | O | Ph | Bn | CH₃-PhCH₂ | H | H | CH₃ |
| 211. | H | HN | O | 4-OH-Ph | H | CH₃-PhCH₂ | H | H | CH₃ |
| 212. | H | HN | O | 4-OH-Ph | Me | CH₃-PhCH₂ | H | H | CH₃ |
| 213. | H | HN | O | 4-OH-Ph | Bn | CH₃-PhCH₂ | H | H | CH₃ |
| 214. | H | O | O | Ph | H | PhCH₂ | H | H | CH₂OH |
| 215. | H | O | O | Ph | H | 4-MeOPhCH₂ | H | H | CH₂OH |
| 216. | H | O | O | Ph | Me | PhCH₂ | H | H | CH₂OH |
| 217. | H | O | O | Ph | Bn | PhCH₂ | H | H | CH₂OH |
| 218. | H | O | O | 4-HO-Ph | H | PhCH₂ | H | H | CH₂OH |
| 219. | H | O | O | 4-HO-Ph | Me | PhCH₂ | H | H | CH₂OH |
| 220. | H | O | O | 4-HO-Ph | Bn | PhCH₂ | H | H | CH₂OH |
| 221. | H | O | O | Ph | H | HOCH₂ | H | H | CH₂OH |
| 222. | H | O | O | Ph | Me | HOCH₂ | H | H | CH₂OH |
| 223. | H | O | O | Ph | Bn | HOCH₂ | H | H | CH₂OH |
| 224. | H | O | O | 4-HO-Ph | H | HOCH₂ | H | H | CH₂OH |
| 225. | H | O | O | 4-HO-Ph | Me | HOCH₂ | H | H | CH₂OH |
| 226. | H | O | O | 4-HO-Ph | Bn | HOCH₂ | H | H | CH₂OH |
| 227. | H | O | O | Ph | H | Bn(HOH₂C)CH | H | H | CH₂OH |
| 228. | H | O | O | Ph | Me | Bn(HOH₂C)CH | H | H | CH₂OH |
| 229. | H | O | O | Ph | Bn | Bn(HOH₂C)CH | H | H | CH₂OH |
| 230. | H | O | O | 4-HO-Ph | H | Bn(HOH₂C)CH | H | H | CH₂OH |
| 231. | H | O | O | 4-HO-Ph | Me | Bn(HOH₂C)CH | H | H | CH₂OH |
| 232. | H | O | O | 4-HO-Ph | Bn | Bn(HOH₂C)CH | H | H | CH₂OH |
| 233. | H | HN | O | Ph | H | PhCH₂ | H | H | H |
| 234. | H | HN | O | Ph | H | 4-MeO-PhCH₂ | H | H | H |
| 235. | H | HN | O | Ph | Me | PhCH₂ | H | H | H |
| 236. | H | HN | O | Ph | Bn | PhCH₂ | H | H | H |
| 237. | H | HN | O | 4-OH-Ph | H | PhCH₂ | H | H | H |
| 238. | H | HN | O | 4-OH-Ph | Me | PhCH₂ | H | H | H |
| 239. | H | HN | O | 4-OH-Ph | Bn | PhCH₂ | H | H | H |
| 240. | H | HN | O | Ph | H | HOCH₂ | H | H | H |
| 241. | H | HN | O | Ph | Me | HOCH₂ | H | H | H |
| 242. | H | HN | O | Ph | Bn | HOCH₂ | H | H | H |
| 243. | H | HN | O | 4-OH-Ph | H | HOCH₂ | H | H | H |
| 244. | H | HN | O | 4-OH-Ph | Me | HOCH₂ | H | H | H |
| 245. | H | HN | O | 4-OH-Ph | Bn | HOCH₂ | H | H | H |
| 246. | H | HN | O | Ph | H | Bn(HOH₂C)CH | H | H | H |
| 247. | H | HN | O | Ph | Me | Bn(HOH₂C)CH | H | H | H |
| 248. | H | HN | O | Ph | Bn | Bn(HOH₂C)CH | H | H | H |
| 249. | H | HN | O | 4-OH-Ph | H | Bn(HOH₂C)CH | H | H | H |
| 250. | H | HN | O | 4-OH-Ph | Me | Bn(HOH₂C)CH | H | H | H |
| 251. | H | HN | S | Ph | H | PhCH₂ | H | H | H |
| 252. | H | HN | S | Ph | H | 4-MeO-PhCH₂ | H | H | H |
| 253. | H | HN | S | Ph | Me | PhCH₂ | H | H | H |
| 254. | H | HN | S | Ph | Bn | PhCH₂ | H | H | H |
| 255. | H | HN | S | 4-OH-Ph | H | PhCH₂ | H | H | H |
| 256. | H | HN | S | 4-OH-Ph | Me | PhCH₂ | H | H | H |
| 257. | H | HN | S | 4-OH-Ph | Bn | PhCH₂ | H | H | H |
| 258. | H | HN | S | Ph | H | HOCH₂ | H | H | H |
| 259. | H | HN | S | Ph | Me | HOCH₂ | H | H | H |
| 260. | H | HN | S | Ph | Bn | HOCH₂ | H | H | H |
| 261. | H | HN | S | 4-OH-Ph | H | HOCH₂ | H | H | H |
| 262. | H | HN | S | 4-OH-Ph | Me | HOCH₂ | H | H | H |
| 263. | H | HN | S | 4-OH-Ph | Bn | HOCH₂ | H | H | H |
| 264. | H | HN | S | Ph | H | Bn(HOH₂C)CH | H | H | H |
| 265. | H | HN | S | Ph | Me | Bn(HOH₂C)CH | H | H | H |
| 266. | H | HN | S | Ph | Bn | Bn(HOH₂C)CH | H | H | H |
| 267. | H | HN | S | 4-OH-Ph | H | Bn(HOH₂C)CH | H | H | H |
| 268. | H | HN | S | 4-OH-Ph | Me | Bn(HOH₂C)CH | H | H | H |

6. Process for the preparation of compounds of formula (I) according to Claim 1 wherein a compound of formula (II) 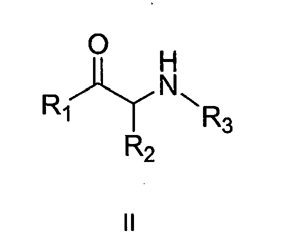 wherein R₁, R₂, R₃, are as above defined
is reacted with a compound of formula (III) 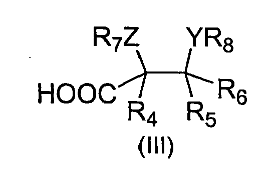 wherein R₄, R₅, R₆, Y and Z are as above defined and R₇ R₈ represent H or suitable protecting groups, (Pg) which can be same or different, cyclic or acyclic, and which can be cleaved in acidic conditions, in order to give a compound of formula (IV) 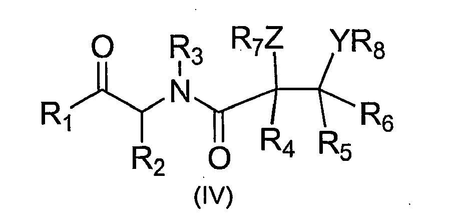 wherein the substituents have the meaning as above, which is cyclised to a compound of formula (I) by action of an acid.

7. Process according to Claim 6 wherein the first step is performed in an aprotic polar solvent at a temperature comprised between 0 - 100°C for 1 - 24 hours.

8. Process according to Claim 7 wherein the reaction is performed in the presence of a coupling agent.

9. Process according to Claim 6 wherein the second step is performed in the presence of a strong acid at a temperature of 0°-150°C for 15min - 24 hours

10. Process according to Claim 9 wherein the acid is chosen in the group consisting of: sulphuric acid adsorbed on silica gel, p-toluen sulphonic acid, trifluoroacetic acid, trifluorometansulphonic acid.

## Patentansprüche

1. Heterobizyklische Derivate der allgemeinen Formel (I): 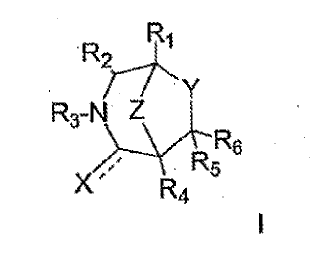 wobei:
R₁ aus der Gruppe ausgewählt ist, die aus C₁₋₈-Alkyl, C₂₋₈-Alkenyl, C₂₋₈-Alkinyl, Cycloalkyl, Aryl, Heterozyklus, Aryl-C₁₋₈-Alkyl; Heterozyklus-C₁₋₈-Alkyl; RR'N-C₁₋₈-Alkyl, RR'N-Aryl, RO-Aryl, R(O)C-Aryl, RO(O)C-Aryl, RR'N(O)C-Aryl, (P)-W-NR-Aryl, (P)-W-O-Aryl, (P)-W-C(O)O-Aryl, (P)-W-O(O)C-Aryl, (P)-W-C(O)RN-Aryl, (P)-W-NR(O)C-Aryl besteht;
R₂ aus der Gruppe ausgewählt ist, die aus H, C₁₋₈-Alkyl, C₂₋₈-Alkenyl, C₂₋₈-Alkinyl, Cycloalkyl, Aryl, Aryl-C₁₋₈-Alkyl; Heterozyklus-C₁₋₈-Alkyl; Amino-C₁₋₈-Alkyl, Aminoaryl, C₁₋₈-Alkyloxyaryl, Hydroxyaryl, Carboxyaryl, Carboalkyloxyaryl, Alkylcarbamoylaryl, -(Seitenkette), -(Seitenkette)-W-(P) besteht, oder
R₁ und R₂ zusammengenommen ein C₁₋₄-Alkyl, C₂₋₄-Alkenyl, Cycloalkyl, benzokondensiertes Cycloalkyl sind, um eine Brücke von 3, 4, 5, 6 Gliedern zu bilden;
R₃ aus der Gruppe ausgewählt ist, die aus H, C₁₋₈-Alkyl, C₂₋₈-Alkenyl, C₂₋₈-Alkinyl, Cycloalkyl, Aryl, Aryl-C₁₋₈-Alkyl; Heterozyklus-C₁₋₈-Alkyl; RR'NC₁₋₈-Alkyl, RR'Naryl, RO-C₁₋₈-Alkyl, RO(O)C-C₁₋₈-Alkyl, R(O)C-C₁₋₈-Alkyl, RC(O)O-C₁₋₈-Alkyl, RC(O)N(R)C₁₋₈-Alkyl RO-Aryl, RO(O)C-Aryl, R(O)C-Aryl RC(O)O-Aryl, RC(O)N(R)-Aryl, -CH-(Aminosäure-Seitenkette)-CO₂R, -CH-(Aminosäure-Seitenkette)-C(O)NR, -CH-(Aminosäure-Seitenkette)-C(O)O-W-(P), -CH-(Aminosäure-Seitenkette)-C(O)N(R)-W-(P), CH(CO₂R)-Aminosäure-Seitenkette-W-(P), CH(CONRR')-Aminosäure-Seitenkette-W-(P), einer Schutzgruppe besteht;
R₄ und R₅, gleich oder verschieden, aus der Gruppe ausgewählt sind, die aus H, C₁₋₈-Alkyl, C₂₋₈-Alkenyl, C₂₋₈-Alkinyl, Cycloalkyl, Aryl, Heterozyklus, Aryl-C₁₋₈-Alkyl; Heterozyklus-C₁₋₈-Alkyl besteht;
R₆ aus der Gruppe ausgewählt ist, die aus H, C₁₋₈-Alkyl, C₂₋₈-Alkenyl, C₂₋₈-Alkinyl, Cycloalkyl, Aryl, Aryl-C₁₋₈-Alkyl, Heterozyklus, Heterozyklus-C₁₋₈-Alkyl; -C(O)R, -C(O)OR, -C(O)NRR', CH₂OR, CH₂NRR', -C(O)NH-CH-(Aminosäure-Seitenkette)-C(O)OR, -C(O)O-W-(P), -C(O)N(R)-W-(P), -CH₂O-W-(P), -CH₂N(R)-W-(P) besteht;
R und R', gleich oder verschieden, aus der Gruppe ausgewählt sind, die aus H, C₁₋₈-Alkyl, C₂₋₈-Alkenyl, C₂₋₈-Alkinyl, Cycloalkyl, Aryl, Heterozyklus, Aryl-C₁₋₈-Alkyl; einer Schutzgruppe, -C(O)CH-(Aminosäure-Seitenkette)-NHR, -NH-CH-(Aminosäure-Seitenkette)-COOR, -CH-(Aminosäure-Seitenkette)-COOR besteht;
P ein Harz ist, entweder löslich oder an einen festen Träger gebunden;
W ein Linker ist;
X O, S ist, wenn α eine Doppelbindung ist, oder X H ist, wenn α eine Einfachbindung ist,
Y und Z, gleich oder verschieden, O, S, SO, SO₂, N-R sind, wobei R wie oben definiert ist;
wobei der Ausdruck Cykloalkyl repräsentiert: Cyclopropan, Cyclobutan, Cyclopentan, Cyclohexan, Cycloheptan, Cyclooctan, Norbornan, Camphan, Adamantin; der Ausdruck Aryl Phenyl-, Biphenyl- und Naphthylgruppen- spezifiziert, die mit einem oder zwei Resten substituiert sind, welche aus den Gruppen gewählt sind, die aus Halogen, Cyano, Nitro, C₁₋₆-Alkyl bestehen; der Ausdruck Heterozyklus Pyridin, Imidazol, Pyrrol, Indol, Triazole, Pyrrolidin, Piperidin repräsentiert, und sich der Ausdruck Schutzgruppe auf bekannte Gruppen bezieht, die eine Reaktion oder Bindung von Sauerstoff, Stickstoff, Carbonsäuren, Thiolen, Alkoholen, Aminen und dergleichen verhindern können;
die oben genannten Alkyl-, Alkenyl-, Alkinyl-, Cycloalkyl-, Aryl- und heterozyklischen Gruppen gegebenenfalls substituiert sind, mit der Maßgabe, dass:
wenn: Z = Y = X = O, R₂= R₄ = R₅ = H, R₃ = CH₃ und R₆ = Ph, dann R₁ nicht Ph ist;
und
wenn X = R₂ = H und Y = Z = O, dann zumindest eines von R₄, R₅ oder R₆ nicht H ist.

2. Heterobizyklische Derivate nach Anspruch 1, wobei:
das Harz P ein polymerer Stoff ist, welcher in den Lösungsmitteln löslich ist, die üblicherweise in der organischen Synthese verwendet werden, oder an einen festen Träger gebunden ist;
der feste Träger (bei Raumtemperatur) ein Feststoff ist, an dem Ausgangsharzmaterialien (reaktive Gruppen) gebunden sein können;
W ein Molekül ist, das das Harz P an die Reagenzien und die Produkte der Formel (I) binden kann;
Aminosäure-Seitenkette die Seitenkettenreste der natürlich vorkommenden L- oder D-Aminosäuren oder der nicht natürlich vorkommenden Aminosäuren bedeutet;
und die weiteren Substituenten sind, wie in dem Anspruch 1 definiert.

3. Heterobizyklische Derivate nach Anspruch 2, wobei:
das Harz ein polymerer Stoff ist, der mit einer NH₂-Gruppe oder einer Hydroxylgruppe derivatisiert ist, welche gegebenenfalls an ein festes Trägermaterial gebunden ist, das aus Polyethylen- und Polystyrolverbindungen und verwandten inerten Polymerverbindungen ausgewählt ist;
die Aminosäure-Seitenkette die Seitenkette einer natürlich oder nicht natürlich vorkommenden Aminosäure ist und die weiteren Substituenten sind, wie in Anspruch 1 definiert.

4. Heterobizyklische Derivate nach Anspruch 3, wobei die nicht natürlich vorkommenden Aminosäuren aus Norleucin (Nle), Norvalin (NVa), β-Alanin, L- oder D-α-Phenylglycin und dergleichen ausgewählt sind und die weiteren Substituenten sind, wie in Anspruch 1 beschrieben.

5. Heterobizyklische Derivate nach Anspruch 4, wiedergegeben durch die folgenden Formeln:
| Verb. | X | Z | Y | R₁ | R₂ | R₃ | R₄ | R₅ | R₆ |
|---|---|---|---|---|---|---|---|---|---|
| 1. | O | O | O | Ph | H | PhCH₂ | H | H | COOH |
| 2. | O | O | O | 4-HO-Ph | H | PhCH₂ | H | H | COOH |
| 3. | O | O | O | 4-O₂N-Ph | H | PhCH₂ | H | H | COOH |
| 4. | O | O | O | 4-H₂N-Ph | H | PhCH₂ | H | H | COOH |
| 5. | O | O | O | 4-MeO(O)C-Ph | H | PhCH₂ | H | H | COOH |
| 6. | O | O | O | 4-Me-Ph | H | PhCH₂ | H | H | COOH |
| 7. | O | O | O | 4-MeO-Ph | H | PhCH₂ | H | H | COOH |
| 8. | O | O | O | 4-Cl-Ph | H | PhCH₂ | H | H | COOH |
| 9. | O | O | O | 4-Br-Ph | H | PhCH₂ | H | H | COOH |
| 10. | O | O | O | 2-HO-Ph | H | PhCH₂ | H | H | COOH |
| 11. | O | O | O | 2-O₂N-Ph | H | PhCH₂ | H | H | COOH |
| 12. | O | O | O | 2-H₂N-Ph | H | PhCH₂ | H | H | COOH |
| 13. | O | O | O | 2-MeO(O)C-Ph | H | PhCH₂ | H | H | COOH |
| 14. | O | O | O | 2-Me-Ph | H | PhCH₂ | H | H | COOH |
| 15. | O | O | O | 2-MeO-Ph | H | PhCH₂ | H | H | COOH |
| 16. | O | O | O | 2-Cl-Ph | H | PhCH₂ | H | H | COOH |
| 17. | O | O | O | 2-Br-Ph | H | PhCH₂ | H | H | COOH |
| 18. | O | O | O | 2-Nafthyl | H | PhCH₂ | H | H | COOH |
| 19. | O | O | O | 2-Thienyl | H | PhCH₂ | H | H | COOH |
| 20. | O | O | O | 4-Biphenyl | H | PhCH₂ | H | H | COOH |
| 21. | O | O | O | Ph | H | Me | H | H | COOH |
| 22. | O | O | O | Ph | H | CH₃(CH₂)₂ | H | H | COOH |
| 23. | O | O | O | Ph | H | Cyclohexyl | H | H | COOH |
| 24. | O | O | O | Ph | H | Allyl | H | H | COOH |
| 25. | O | O | O | Ph | H | Ph | H | H | COOH |
| 26. | O | O | O | Ph | H | 4-HO-Ph | H | H | COOH |
| 27. | O | O | O | Ph | H | 4-O₂N-Ph | H | H | COOH |
| 28. | O | O | O | Ph | H | 4-MeO₂C-Ph | H | H | COOH |
| 29. | O | O | O | Ph | H | 4-Me-Ph | H | H | COOH |
| 30. | O | O | O | Ph | H | 4-MeO-Ph | H | H | COOH |
| 31. | O | O | O | Ph | H | 4-Cl-Ph | H | H | COOH |
| 32. | O | O | O | Ph | H | 4-Br-Ph | H | H | COOH |
| 33. | O | O | O | Ph | H | 2-HO-Ph | H | H | COOH |
| 34. | O | O | O | Ph | H | 2-O₂N-Ph | H | H | COOH |
| 35. | O | O | O | Ph | H | 2-MeO₂C-Ph | H | H | COOH |
| 36. | O | O | O | Ph | H | 2-Me-Ph | H | H | COOH |
| 37. | O | O | O | Ph | H | 2-MeO-Ph | H | H | COOH |
| 38. | O | O | O | Ph | H | 2-Cl-Ph | H | H | COOH |
| 39. | O | O | O | Ph | H | 2-Br-Ph | H | H | COOH |
| 40. | O | O | O | Ph | H | 2-Naphthyl | H | H | COOH |
| 41. | O | O | O | Ph | H | 2-Thienyl | H | H | COOH |
| 42. | O | O | O | Ph | H | 4-Biphenyl | H | H | COOH |
| 43. | O | O | O | Ph | H | 4-MeO₂C-PhCH₂ | H | H | COOH |
| 44. | O | O | O | Ph | H | 4-Me-PhCH₂ | H | H | COOH |
| 45. | O | O | O | Ph | H | 4-MeOPhCH₂ | H | H | COOH |
| 46. | O | O | O | Ph | H | 4-Cl-PhCH₂ | H | H | COOH |
| 47. | O | O | O | Ph | H | 4-Br-PhCH₂ | H | H | COOH |
| 48. | O | O | O | Ph | H | 2-HO-PhCH₂ | H | H | COOH |
| 49. | O | O | O | Ph | H | 2-O₂N-PhCH₂ | H | H | COOH |
| 50. | O | O | O | Ph | H | 2-MeO₂C-PhCH₂ | H | H | COOH |
| 51. | O | O | O | Ph | H | 2-Me-PhCH₂ | H | H | COOH |
| 52. | O | O | O | Ph | H | 2-MeO-PhCH₂ | H | H | COOH |
| 53. | O | O | O | Ph | H | 2-CI-PhCH₂ | H | H | COOH |
| 54. | O | O | O | Ph | H | 2-Br-PhCH₂ | H | H | COOH |
| 55. | O | O | O | 4-HO-Ph | H | 4-HO-PhCH₂ | H | H | COOH |
| 56. | O | O | O | 4-HO-Ph | H | 4-O₂N-PhCH₂ | H | H | COOH |
| 57. | O | O | O | 4-HO-Ph | H | 4-MeO₂C-PhCH₂ | H | H | COOH |
| 58. | O | O | O | 4-HO-Ph | H | 4-Me-PhCH₂ | H | H | COOH |
| 59. | O | O | O | 4-HO-Ph | H | 4-MeOPhCH₂ | H | H | COOH |
| 60. | O | O | O | 4-HO-Ph | H | 4-Cl-PhCH₂ | H | H | COOH |
| 61. | O | O | O | 4-HO-Ph | H | 4-Br-PhCH₂ | H | H | COOH |
| 62. | O | O | O | 4-HO-Ph | H | 2-HO-PhCH₂ | H | H | COOH |
| 63. | O | O | O | 4-HO-Ph | H | 2-O₂N-PhCH₂ | H | H | COOH |
| 64. | O | O | O | 4-HO-Ph | H | 2-MeO₂C-PhCH₂ | H | H | COOH |
| 65. | O | O | O | 4-HO-Ph | H | 2-Me-PhCH₂ | H | H | COOH |
| 66. | O | O | O | 4-HO-Ph | H | 2-MeO-PhCH₂ | H | H | COOH |
| 67. | O | O | O | 4-HO-Ph | H | 2-Cl-PhCH₂ | H | H | COOH |
| 68. | O | O | O | 4-HO-Ph | H | 2-Br-PhCH₂ | H | H | COOH |
| 69. | O | O | O | 4-HO-Ph | H | Me | H | H | COOH |
| 70. | O | O | O | 4-HO-Ph | H | CH₃(CH₂)₂ | H | H | COOH |
| 71. | O | O | O | 4-HO-Ph | H | Cyclohexyl | H | H | COOH |
| 72. | O | O | O | 4-HO-Ph | H | Allyl | H | H | COOH |
| 73. | O | O | O | Ph | H | HO₂C-CH₂ | H | H | COOH |
| 74. | O | O | O | Ph | H | Bn(HO₂C)CH | H | H | COOH |
| 75. | O | O | O | Ph | H | HOCH₂(HO₂C)CH | H | H | COOH |
| 76. | O | O | O | Ph | H | CH₃(HO)CH(HO₂C)CH | H | H | COOH |
| 77. | O | O | O | Ph | H | MeS(CH₂)₂(HO₂C)CH | H | H | COOH |
| 78. | O | O | O | Ph | H | H₂N(CH₂)₃(HO₂C)CH | H | H | COOH |
| 79. | O | O | O | Ph | H | HO₂CCH₂(HO₂C)CH | H | H | COOH |
| 80. | O | O | O | Ph | H | Imidazol-CH₂(HO₂C)CH | H | H | COOH |
| 81. | O | O | O | Ph | H | Indol-CH₂(HO₂C)CH | H | H | COOH |
| 82. | O | O | O | 4-HO-Ph | H | HO₂C-CH₂ | H | H | COOH |
| 83. | O | O | O | 4-HO-Ph | H | Me(HO₂C)CH | H | H | COOH |
| 84. | O | O | O | 4-HO-Ph | H | (CH₃)₂CH(HO₂C)CH | H | H | COOH |
| 85. | O | O | O | 4-HO-Ph | H | Bn(HO₂C)CH | H | H | COOH |
| 86. | O | O | O | 4-HO-Ph | H | HOCH₂(HO₂C)CH | H | H | COOH |
| 87. | O | O | O | 4-HO-Ph | H | CH₃(HO)CH(HO₂C)CH | H | H | COOH |
| 88. | O | O | O | 4-HO-Ph | H | MeS(CH₂)₂(HO₂C)CH | H | H | COOH |
| 89. | O | O | O | 4-HO-Ph | H | H₂N(CH₂)₃(HO₂C)CH | H | H | COOH |
| 90. | O | O | O | 4-HO-Ph | H | HO₂CCH₂(HO₂C)CH | H | H | COOH |
| 91. | O | O | O | 4-HO-Ph | H | Imidazol-CH₂(HO₂C)CH | H | H | COOH |
| 92. | O | O | O | 4-HO-Ph | H | Indol-CH₂(HO₂C)CH | H | H | COOH |
| 93. | O | O | O | Ph | Me | PhCH₂ | H | H | COOH |
| 94. | O | O | O | 4-HO-Ph | Me | PhCH₂ | H | H | COOH |
| 95. | O | O | O | Ph | Bn | PhCH₂ | H | H | COOH |
| 96. | O | O | O | 4-HO-Ph | Bn | PhCH₂ | H | H | COOH |
| 97. | O | O | O | Ph | Me | HO₂C-CH₂ | H | H | COOH |
| 98. | O | O | O | 4-HO-Ph | Me | HO₂C-CH₂ | H | H | COOH |
| 99. | O | O | O | Ph | Bn | HO₂C-CH₂ | H | H | COOH |
| 100. | O | O | O | 4-HO-Ph | Bn | HO₂C-CH₂ | H | H | COOH |
| 101. | O | O | O | Ph | Me | Bn(HO₂C)CH | H | H | COOH |
| 102. | O | O | O | 4-HO-Ph | Me | Bn(HO₂C)CH | H | H | COOH |
| 103. | O | HN | O | Ph | H | PhCH₂ | H | H | CH₃ |
| 104. | O | HN | O | Ph | Me | PhCH₂ | H | H | CH₃ |
| 105. | O | HN | O | Ph | Bn | PhCH₂ | H | H | CH₃ |
| 106. | O | HN | O | 4-OH-Ph | H | PhCH₂ | H | H | CH₃ |
| 107. | O | HN | O | 4-OH-Ph | Me | PhCH₂ | H | H | CH₃ |
| 108. | O | HN | O | 4-OH-Ph | Bn | PhCH₂ | H | H | CH₃ |
| 109. | O | HN | O | Ph | H | Ph | H | H | CH₃ |
| 110. | O | HN | O | Ph | Me | Ph | H | H | CH₃ |
| 111. | O | HN | O | Ph | Bn | Ph | H | H | CH₃ |
| 112. | O | HN | O | 4-OH-Ph | H | Ph | H | H | CH₃ |
| 113. | O | HN | O | 4-OH-Ph | Me | Ph | H | H | CH₃ |
| 114. | O | HN | O | 4-OH-Ph | Bn | Ph | H | H | CH₃ |
| 115. | O | HN | O | Ph | H | CH₃-Ph | H | H | CH₃ |
| 116. | O | HN | O | Ph | Me | CH₃-Ph | H | H | CH₃ |
| 117. | O | HN | O | Ph | Bn | CH₃-Ph | H | H | CH₃ |
| 118. | O | HN | O | 4-OH-Ph | H | CH₃-Ph | H | H | CH₃ |
| 119. | O | HN | O | 4-OH-Ph | Me | CH₃-Ph | H | H | CH₃ |
| 120. | O | HN | O | 4-OH-Ph | Bn | CH₃-Ph | H | H | CH₃ |
| 121. | O | HN | O | Ph | H | 4-MeO-PhCH₂ | H | H | CH₃ |
| 122. | O | HN | O | Ph | Me | 4-MeO-PhCH₂ | H | H | CH₃ |
| 123. | O | HN | O | Ph | Bn | 4-MeO-PhCH₂ | H | H | CH₃ |
| 124. | O | HN | O | 4-OH-Ph | H | 4-MeO-PhCH₂ | H | H | CH₃ |
| 125. | O | HN | O | 4-OH-Ph | Me | 4-MeO-PhCH₂ | H | H | CH₃ |
| 126. | O | HN | O | 4-OH-Ph | Bn | 4-MeO-PhCH₂ | H | H | CH₃ |
| 127. | O | HN | O | Ph | H | CH₃-PhCH₂ | H | H | CH₃ |
| 128. | O | HN | O | Ph | Me | CH₃-PhCH₂ | H | H | CH₃ |
| 129. | O | HN | O | Ph | Bn | CH₃-PhCH₂ | H | H | CH₃ |
| 130. | O | HN | O | 4-OH-Ph | H | CH₃-PhCH₂ | H | H | CH₃ |
| 131. | O | HN | O | 4-OH-Ph | Me | CH₃-PhCH₂ | H | H | CH₃ |
| 132. | O | HN | O | 4-OH-Ph | Bn | CH₃-PhCH₂ | H | H | CH₃ |
| 133. | O | HN | O | Ph | H | Me | H | H | CH₃ |
| 134. | O | HN | O | Ph | H | CH₃(CH₂)₂ | H | H | CH₃ |
| 135. | O | HN | O | Ph | H | Cyclohexyl | H | H | CH₃ |
| 136. | O | HN | O | Ph | H | Allyl | H | H | CH₃ |
| 137. | O | HN | O | 4-OH-Ph | H | Me | H | H | CH₃ |
| 138. | O | HN | O | 4-OH-Ph | H | CH₃(CH₂)₂ | H | H | CH₃ |
| 139. | O | HN | O | 4-OH-Ph | H | Cyclohexyl | H | H | CH₃ |
| 140. | O | HN | O | 4-OH-Ph | H | Allyl | H | H | CH₃ |
| 141. | O | HN | O | Ph | H | HO₂C-CH₂ | H | H | CH₃ |
| 142. | O | HN | O | Ph | Me | HO₂C-CH₂ | H | H | CH₃ |
| 143. | O | HN | O | Ph | Bn | HO₂C-CH₂ | H | H | CH₃ |
| 144. | O | HN | O | 4-OH-Ph | H | HO₂C-CH₂ | H | H | CH₃ |
| 145. | O | HN | O | 4-OH-Ph | Me | HO₂C-CH₂ | H | H | CH₃ |
| 146. | O | HN | O | 4-OH-Ph | Bn | HO₂C-CH₂ | H | H | CH₃ |
| 147. | O | HN | O | Ph | H | Bn(HO₂C)CH | H | H | CH₃ |
| 148. | O | HN | O | Ph | Me | Bn(HO₂C)CH | H | H | CH₃ |
| 149. | O | HN | O | Ph | Bn | Bn(HO₂C)CH | H | H | CH₃ |
| 150. | 0 | HN | O | 4-OH-Ph | H | Bn(HO₂C)CH | H | H | CH₃ |
| 151. | 0 | HN | O | 4-OH-Ph | Me | Bn(HO₂C)CH | H | H | CH₃ |
| 152. | 0 | HN | O | 4-OH-Ph | Bn | Bn(HO₂C)CH | H | H | CH₃ |
| 153. | H | O | O | Ph | H | PhCH₂ | H | H | COOH |
| 154. | H | O | O | Ph | Me | PhCH₂ | H | H | COOH |
| 155. | H | O | O | Ph | Bn | PhCH₂ | H | H | COOH |
| 156. | H | O | O | 4-HO-Ph | H | PhCH₂ | H | H | COOH |
| 157. | H | O | O | 4-HO-Ph | Me | PhCH₂ | H | H | COOH |
| 158. | H | O | O | 4-HO-Ph | Bn | PhCH₂ | H | H | COOH |
| 159. | H | O | O | Ph | H | HO₂C-CH₂ | H | H | COOH |
| 160. | H | O | O | Ph | Me | HO₂C-CH₂ | H | H | COOH |
| 161. | H | O | O | Ph | Bn | HO₂C-CH₂ | H | H | COOH |
| 162. | H | O | O | 4-HO-Ph | H | HO₂C-CH₂ | H | H | COOH |
| 163. | H | O | O | 4-HO-Ph | Me | HO₂C-CH₂ | H | H | COOH |
| 164. | H | O | O | 4-HO-Ph | Bn | HO₂C-CH₂ | H | H | COOH |
| 165. | H | O | O | Ph | H | Bn(HO₂C)CH | H | H | COOH |
| 166. | H | O | O | Ph | Me | Bn(HO₂C)CH | H | H | COOH |
| 167. | H | O | O | Ph | Bn | Bn(HO₂C)CH | H | H | COOH |
| 168. | H | O | O | 4-HO-Ph | H | Bn(HO₂C)CH | H | H | COOH |
| 169. | H | O | O | 4-HO-Ph | Me | Bn(HO₂C)CH | H | H | COOH |
| 170. | H | O | O | 4-HO-Ph | Bn | Bn(HO₂C)CH | H | H | COOH |
| 171. | H | HN | O | Ph | H | PhCH₂ | H | H | CH₃ |
| 172. | H | HN | O | Ph | H | 4-MeO-PhCH₂ | H | H | CH₃ |
| 173. | H | HN | O | Ph | Me | PhCH₂ | H | H | CH₃ |
| 174. | H | HN | O | Ph | Bn | PhCH₂ | H | H | CH₃ |
| 175. | H | HN | O | 4-OH-Ph | H | PhCH₂ | H | H | CH₃ |
| 176. | H | HN | O | 4-OH-Ph | Me | PhCH₂ | H | H | CH₃ |
| 177. | H | HN | O | 4-OH-Ph | Bn | PhCH₂ | H | H | CH₃ |
| 178. | H | HN | O | Ph | H | HO₂C-CH₂ | H | H | CH₃ |
| 179. | H | HN | O | Ph | Me | HO₂C-CH₂ | H | H | CH₃ |
| 180. | H | HN | O | Ph | Bn | HO₂C-CH₂ | H | H | CH₃ |
| 181. | H | HN | O | 4-OH-Ph | H | HO₂C-CH₂ | H | H | CH₃ |
| 182. | H | HN | O | 4-OH-Ph | Me | HO₂C-CH₂ | H | H | CH₃ |
| 183. | H | HN | O | 4-OH-Ph | Bn | HO₂C-CH₂ | H | H | CH₃ |
| 184. | H | HN | O | Ph | H | Bn(HO₂C)CH | H | H | CH₃ |
| 185. | H | HN | O | Ph | Me | Bn(HO₂C)CH | H | H | CH₃ |
| 186. | H | HN | O | Ph | Bn | Bn(HO₂C)CH | H | H | CH₃ |
| 187. | H | HN | O | 4-OH-Ph | H | Bn(HO₂C)CH | H | H | CH₃ |
| 188. | H | HN | O | 4-OH-Ph | Me | Bn(HO₂C)CH | H | H | CH₃ |
| 189. | H | HN | O | 4-OH-Ph | Bn | Bn(HO₂C)CH | H | H | CH₃ |
| 190. | H | HN | O | Ph | H | Ph | H | H | CH₃ |
| 191. | H | HN | O | Ph | Me | Ph | H | H | CH₃ |
| 192. | H | HN | O | Ph | Bn | Ph | H | H | CH₃ |
| 193. | H | HN | O | 4-OH-Ph | H | Ph | H | H | CH₃ |
| 194. | H | HN | O | 4-OH-Ph | Me | Ph | H | H | CH₃ |
| 195. | H | HN | O | 4-OH-Ph | Bn | Ph | H | H | CH₃ |
| 196. | H | HN | O | Ph | H | CH₃-Ph | H | H | CH₃ |
| 197. | H | HN | O | Ph | Me | CH₃-Ph | H | H | CH₃ |
| 198. | H | HN | O | Ph | Bn | CH₃-Ph | H | H | CH₃ |
| 199. | H | HN | O | 4-OH-Ph | H | CH₃-Ph | H | H | CH₃ |
| 200. | H | HN | O | 4-OH-Ph | Me | CH₃-Ph | H | H | CH₃ |
| 201. | H | HN | O | 4-OH-Ph | Bn | CH3-Ph | H | H | CH₃ |
| 202. | H | HN | O | Ph | H | 4-MeO-PhCH₂ | H | H | CH₃ |
| 203. | H | HN | O | Ph | Me | 4-MeO-PhCH₂ | H | H | CH₃ |
| 204. | H | HN | O | Ph | Bn | 4-MeO-PhCH₂ | H | H | CH₃ |
| 205. | H | HN | O | 4-OH-Ph | H | 4-MeO-PhCH₂ | H | H | CH₃ |
| 206. | H | HN | O | 4-OH-Ph | Me | 4-MeO-PhCH₂ | H | H | CH₃ |
| 207. | H | HN | O | 4-OH-Ph | Bn | 4-MeO-PhCH₂ | H | H | CH₃ |
| 208. | H | HN | O | Ph | H | CH₃-PhCH₂ | H | H | CH₃ |
| 209. | H | HN | O | Ph | Me | CH₃-PhCH₂ | H | H | CH₃ |
| 210. | H | HN | O | Ph | Bn | CH₃-PhCH₂ | H | H | CH₃ |
| 211. | H | HN | O | 4-OH-Ph | H | CH₃-PhCH₂ | H | H | CH₃ |
| 212. | H | HN | O | 4-OH-Ph | Me | CH₃-PhCH₂ | H | H | CH₃ |
| 213. | H | HN | O | 4-OH-Ph | Bn | CH₃-PhCH₂ | H | H | CH₃ |
| 214. | H | O | O | Ph | H | PhCH₂ | H | H | CH₂OH |
| 215. | H | O | O | Ph | H | 4-MeOPhCH₂ | H | H | CH₂OH |
| 216. | H | O | O | Ph | Me | PhCH₂ | H | H | CH₂OH |
| 217. | H | O | O | Ph | Bn | PhCH₂ | H | H | CH₂OH |
| 218. | H | O | O | 4-HO-Ph | H | PhCH₂ | H | H | CH₂OH |
| 219. | H | O | O | 4-HO-Ph | Me | PhCH₂ | H | H | CH₂OH |
| 220. | H | O | O | 4-HO-Ph | Bn | PhCH₂ | H | H | CH₂OH |
| 221. | H | O | O | Ph | H | HOCH₂ | H | H | CH₂OH |
| 222. | H | O | O | Ph | Me | HOCH₂ | H | H | CH₂OH |
| 223. | H | O | O | Ph | Bn | HOCH₂ | H | H | CH₂OH |
| 224. | H | O | O | 4-HO-Ph | H | HOCH₂ | H | H | CH₂OH |
| 225. | H | O | O | 4-HO-Ph | Me | HOCH₂ | H | H | CH₂OH |
| 226. | H | O | O | 4-HO-Ph | Bn | HOCH₂ | H | H | CH₂OH |
| 227. | H | O | O | Ph | H | Bn(HOH₂C)CH | H | H | CH₂OH |
| 228. | H | O | O | Ph | Me | Bn(HOH₂C)CH | H | H | CH₂OH |
| 229. | H | O | O | Ph | Bn | Bn(HOH₂C)CH | H | H | CH₂OH |
| 230. | H | O | O | 4-HO-Ph | H | Bn(HOH₂C)CH | H | H | CH₂OH |
| 231. | H | O | O | 4-HO-Ph | Me | Bn(HOH₂C)CH | H | H | CH₂OH |
| 232. | H | O | O | 4-HO-Ph | Bn | Bn(HOH₂C)CH | H | H | CH₂OH |
| 233. | H | HN | O | Ph | H | PhCH₂ | H | H | H |
| 234. | H | HN | O | Ph | H | 4-MeO-PhCH₂ | H | H | H |
| 235. | H | HN | O | Ph | Me | PhCH₂ | H | H | H |
| 236. | H | HN | O | Ph | Bn | PhCH₂ | H | H | H |
| 237. | H | HN | O | 4-OH-Ph | H | PhCH₂ | H | H | H |
| 238. | H | HN | O | 4-OH-Ph | Me | PhCH₂ | H | H | H |
| 239. | H | HN | O | 4-OH-Ph | Bn | PhCH₂ | H | H | H |
| 240. | H | HN | O | Ph | H | HOCH₂ | H | H | H |
| 241. | H | HN | O | Ph | Me | HOCH₂ | H | H | H |
| 242. | H | HN | O | Ph | Bn | HOCH₂ | H | H | H |
| 243. | H | HN | O | 4-OH-Ph | H | HOCH₂ | H | H | H |
| 244. | H | HN | O | 4-OH-Ph | Me | HOCH₂ | H | H | H |
| 245. | H | HN | O | 4-OH-Ph | Bn | HOCH₂ | H | H | H |
| 246. | H | HN | O | Ph | H | Bn(HOH₂C)CH | H | H | H |
| 247. | H | HN | O | Ph | Me | Bn(HOH₂C)CH | H | H | H |
| 248. | H | HN | O | Ph | Bn | Bn(HOH₂C)CH | H | H | H |
| 249. | H | HN | O | 4-OH-Ph | H | Bn(HOH₂C)CH | H | H | H |
| 250. | H | HN | O | 4-OH-Ph | Me | Bn(HOH₂C)CH | H | H | H |
| 251. | H | HN | S | Ph | H | PhCH₂ | H | H | H |
| 252. | H | HN | S | Ph | H | 4-MeO-PhCH₂ | H | H | H |
| 253. | H | HN | S | Ph | Me | PhCH₂ | H | H | H |
| 254. | H | HN | S | Ph | Bn | PhCH₂ | H | H | H |
| 255. | H | HN | S | 4-OH-Ph | H | PhCH₂ | H | H | H |
| 256. | H | HN | S | 4-OH-Ph | Me | PhCH₂ | H | H | H |
| 257. | H | HN | S | 4-OH-Ph | Bn | PhCH₂ | H | H | H |
| 258. | H | HN | S | Ph | H | HOCH₂ | H | H | H |
| 259. | H | HN | S | Ph | Me | HOCH₂ | H | H | H |
| 260. | H | HN | S | Ph | Bn | HOCH₂ | H | H | H |
| 261. | H | HN | S | 4-OH-Ph | H | HOCH₂ | H | H | H |
| 262. | H | HN | S | 4-OH-Ph | Me | HOCH₂ | H | H | H |
| 263. | H | HN | S | 4-OH-Ph | Bn | HOCH₂ | H | H | H |
| 264. | H | HN | S | Ph | H | Bn(HOH₂C)CH | H | H | H |
| 265. | H | HN | S | Ph | Me | Bn(HOH₂C)CH | H | H | H |
| 266. | H | HN | S | Ph | Bn | Bn(HOH₂C)CH | H | H | H |
| 267. | H | HN | S | 4-OH-Ph | H | Bn(HOH₂C)CH | H | H | H |
| 268. | H | HN | S | 4-OH-Ph | Me | Bn(HOH₂C)CH | H | H | H |

6. Verfahren zur Herstellung von Verbindungen der Formel (I) nach Anspruch 1, wobei eine Verbindung der Formel (II) 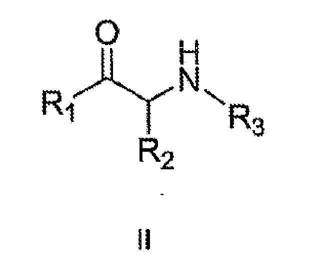 worin R₁, R₂, R₃ wie oben definiert sind, mit einer Verbindung der Formel (III) 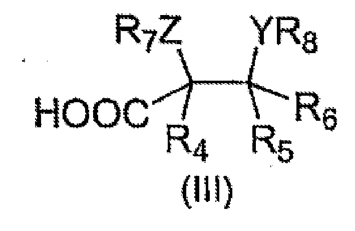 reagiert wird, worin R₄, R₅, R₆ , Y und Z wie oben definiert sind und R₇ und R₈ H oder geeignete Schutzgruppen (Pg) repräsentieren, welche gleiche oder verschiedene, zyklisch oder azyklisch sein können, und die unter saueren Bedingungen abgespalten werden können, um eine Verbindung mit der Formel (IV) 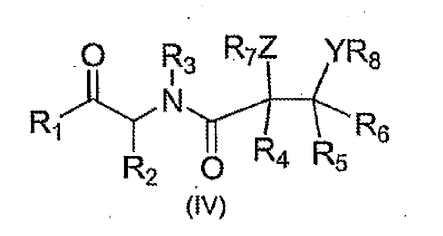 zu ergeben, wobei die Substituenten die vorstehend angegebene Bedeutung aufweisen, wobei diese durch die Wirkung einer Säure zu einer Verbindung der Formel (I) zyklisiert wird.

7. Verfahren nach Anspruch 6, wobei der erste Schritt in einem aprotischen polaren Lösungsmittel bei einer Temperatur zwischen 0 und 100 °C für 1 bis 24 Stunden ausgeführt wird.

8. Verfahren nach Anspruch 7, wobei die Reaktion in der Gegenwart eines Haftvermittlers ausgeführt wird.

9. Verfahren nach Anspruch 6, wobei der zweite Schritt in Gegenwart einer starken Säure bei einer Temperatur zwischen 0 ° und 150 °C für 15 Min. bis 24 Stunden ausgeführt wird.

10. Verfahren nach Anspruch 9, wobei die Säure aus der Gruppe ausgewählt wird, die besteht aus: an Silicagel adsorbierter Schwefelsäure, p-Toluolsulfonsäure, Trifluoressigsäure, Trifluormethansulfonsäure.

## Revendications

1. Dérivés hétérobicycliques de formule générale (1) 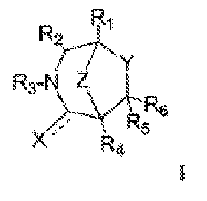 dans laquelle :
R₁ est choisi dans le groupe constitué par les groupes alkyle en C₁₋₈, alcényle en C₂₋₈, alcynyle en C₂₋₈, cycloalkyle, aryle, hétérocycle, aryl(alkyle en C₁₋₈) ; hétérocycle(alkyle en C₁₋₈) ; RR'N-(alkyle en C₁₋₈), RR'N-aryle, RO-aryle, R(O)C-aryle, RO(O)C-aryle, RR'N(O)C-aryle, (P)-W-NR-aryle, (P)-W-O-aryle, (P)-W-C(O)O-aryle, (P)-W-O(O)C-aryle, (P)-W-C(O)RN-aryle, (P)-W-NR(O)C-aryle ;
R₂, est choisi dans le groupe constitué par H, les groupes alkyle en C₁₋₈, alcényle en C₂₋₈, alcynyle en C₂₋₈, cycloalkyle, aryle, aryl(alkyle en C₁₋₈) ; hétérocycle(alkyl en C₁₋₈); amino(alkyle en C₁₋₈), aminoaryle, (alkyloxy en C₁₋₈)aryle, hydroxyaryle, carboxyaryle, carboalkyloxyaryle, alkylcarbamoylaryle, -(chaîne latérale), -(chaîne latérale)-W-(P) ; ou
R₁ et R₂ forment ensemble un groupe alkyle en C_{1 à 4}, alcényle en C_{2 à 4}, cycloalkyle, cycloalkyle benzocondensé, pour former un pont de 3, 4, 5 ou 6 chaînons ;
R₃ est choisi dans le groupe constitué par H, les groupes alkyle en C₁₋₈, alcényle en C₂₋₈, alcynyle en C₂₋₈, cycloalkyle, aryle, aryl(alkyle en C₁₋₈) ; hétérocycle(alkyle en C₁₋₈) ; RR'N(alkyle en C₁₋₈), RR'Naryle, RO-(alkyle en C₁₋₈), RO(O)C-(alkyle en C₁₋₈), R(O)C-(alkyle en C₁₋₈), RC(O)O-(alkyle en C₁₋₈), RC(O)N(R)(alkyl en C₁₋₈)RO-aryle, RO(O)C-aryle, R(O)C-aryl RC(O)O-aryle, RC(O)N(R)aryle, -CH(chaîne latérale acide aminé)CO₂R, -CH(chaîne latérale acide aminé)C(O)NR, -CH(chaîne latérale acide aminé)-C(O)O-W-(P), -CH(chaîne latérale acide aminé)-C(O)N(R)-W-(P), CH(CO₂R)-chaîne latérale acide aminé -W-(P), CH(CONRR')- chaîne latérale acide aminé -W-(P), un groupe protecteur ;
R₄ et R₅, identiques ou différents, sont choisis dans le groupe constitué par H, les groupes alkyle en C₁₋₈, alcényle en C₂₋₈, alcynyle en C₂₋₈, cycloalkyle, aryle, aryl(alkyle en C₁₋₈) ; hétérocycle(alkyle en C₁₋₈) ;
R₆ est choisi dans le groupe constitué par H, les groupes alkyle en C₁₋₈, alcényle en C₂₋₈, alcynyle en C₂₋₈, cycloalkyle, aryle, aryl(alkyle en C₁₋₈) ; hétérocycle ; hétérocycle(alkyle en C₁₋₈) ; -C(O)R, -C(O)OR, -C(O)NRR', CH₂OR, CH₂NRR', -C(O)NH-CH(chaîne latérale acide aminé)C(O)OR, - C(O)O-W-(P), -C(O)N(R)-W-(P), -CH₂O-W-(P), -CH₂N(R)-W-(P) ;
R et R', identiques ou différents, sont choisis dans le groupe constitué par H, les groupes alkyle en C₁₋₈, alcényle en C₂₋₈, alcynyle en C₂₋₈, cycloalkyle, aryle, hétérocycle, aryl(alkyle en C₁₋₈) ; hétérocycle(alkyle en C₁₋₈) ; un groupe protecteur,
-C(O)CH-(chaîne latérale acide aminé)-NHR, -NH-CH(chaîne latérale acide aminé)COOR, -CH(chaîne latérale acide aminé)COOR ;
P est une résine, soluble ou liée à un support solide ;
W est un espaceur ;
X est O, S, lorsque *a* est une double liaison, ou X est H et *a* est une liaison simple,
Y et Z, identiques ou différents, sont O, S, SO, SO₂, N-R, où R est comme défini ci-dessus ;
le terme cycloalkyle représentant le cyclopropane, le cyclobutane, le cyclopentane, le cyclohexane, le cycloheptane, le cyclooctane, le norbornane, le camphane, l'adamantine ; le terme aryle désignant les groupes phényle, biphényle et naphtyle substitués par une ou deux radicaux choisis dans le groupe constitué par un atome d'halogène, un groupe cyano, nitro, alkyle en C_{1 à 6} ; le terme hétérocycle répsentant la pyridine, l'imidazole, la pyrrole, l'indole, les triazoles, la pyrrolidine, la pipéridine, et l'expression groupe protecteur se référant à des groupes connus capables d'empêcher une réaction ou une liaison de l'oxygène, de l'azote, des acides carboxyliques, des thiols, des alcools, des amines et similaires ;
lesdits groupes alkyle, alcényle, alcynyle, cycloalkyle, aryle et hétérocycle ci-dessus étant éventuellement substitués
étant entendu que :
lorsque : Z = Y = X = O, R₂ = R₄ = R₅ = H, R₃ = CH₃ et R₆ = Ph, alors R₁ est différent de Ph ; et
lorsque X = R₂ = H et Y = Z = O, alors au moins l'un parmi R₄, R₅ et R₆ est différent de H.

2. Dérivés hétérobicyclique selon la revendication 1, dans lesquels :
la résine P est un matériau polymère soluble dans les solvants couramment utilisé dans les synthèses organiques ou lié à un support solide ;
le support solide est un matériau solide (à température ambiante) auquel des matériaux de résine de départ (groupes réactifs) peuvent être liés ;
W est une molécule capable de lier la résine P aux réactifs et aux produits de formule (I) ;
chaîne latérale acide aminé signifie les fractions de chaîne latérale des acides aminés L ou D présents à l'état naturel ou des acides aminés non présents à l'état naturel ;
et les autres substituants sont comme défini dans la revendication 1.

3. Dérivés d'hétérobicycle selon la revendication 2, dans lesquels :
la résine est un polymère modifié par un groupe -NH₂ ou un groupe hydroxyle éventuellement lié à un matériau de support solide choisi parmi les composés poly(éthylène) et poly(styrène) et les composés polymère inertes analogues ;
la chaîne latérale acide aminé est la chaîne latérale d'un acide aminé présent à l'état naturel ou non présent à l'état naturel et les autres substituants sont comme défini dans la revendication 1.

4. Dérivés d'hétérobicycle selon la revendication 3, dans lesquels les acides aminés non présents à l'état naturel sont choisis parmi la norleucine (Nle), la norvaline (NVa), la β- alanine, l'α-phényl glycine L ou D et similaires et les autres substituants sont comme décrit dans la revendication 1.

5. Dérivés d'hétérobicycle selon la revendication 4, représentés par les formules suivantes :
| Comp. | X | Z | Y | R₁ | R₂ | R₃ | R₄ | R₅ | R₆ |
|---|---|---|---|---|---|---|---|---|---|
| 1. | O | O | O | Ph | H | PhCH₂ | H | H | COOH |
| 2. | O | O | O | 4-HO-Ph | H | PhCH₂ | H | H | COOH |
| 3. | O | O | O | 4-O₂N-Ph | H | PhCH₂ | H | H | COOH |
| 4. | O | O | O | 4-H₂N-Ph | H | PhCH₂ | H | H | COOH |
| 5. | O | O | O | 4-MeO(O)C-Ph | H | PhCH₂ | H | H | COOH |
| 6. | O | O | O | 4-Me-Ph | H | PhCH₂ | H | H | COOH |
| 7. | O | O | O | 4-MeO-Ph | H | PhCH₂ | H | H | COOH |
| 8. | O | O | O | 4-Cl-Ph | H | PhCH₂ | H | H | COOH |
| 9. | O | O | O | 4-Br-Ph | H | PhCH₂ | H | H | COOH |
| 10. | O | O | O | 2-HO-Ph | H | PhCH₂ | H | H | COOH |
| 11. | O | O | O | 2-O₂N-Ph | H | PhCH₂ | H | H | COOH |
| 12. | O | O | O | 2-H₂N-Ph | H | PhCH₂ | H | H | COOH |
| 13. | O | O | O | 2-MeO(O)C-Ph | H | PhCH₂ | H | H | COOH |
| 14. | O | O | O | 2-Me-Ph | H | PhCH₂ | H | H | COOH |
| 15. | O | O | O | 2-MeO-Ph | H | PhCH₂ | H | H | COOH |
| 16. | O | O | O | 2-Cl-Ph | H | PhCH₂ | H | H | COOH |
| 17. | O | O | O | 2-Br-Ph | H | PhCH₂ | H | H | COOH |
| 18. | O | O | O | 2-naphtyl | H | PhCH₂ | H | H | COOH |
| 19. | O | O | O | 2-thiényl | H | PhCH₂ | H | H | COOH |
| 20. | O | O | O | 4-biphényl | H | PhCH₂ | H | H | COOH |
| 21. | O | O | O | Ph | H | Me | H | H | COOH |
| 22. | O | O | O | Ph | H | CH₃(CH₂)₂ | H | H | COOH |
| 23. | O | O | O | Ph | H | cyclohexyl | H | H | COOH |
| 24. | O | O | O | Ph | H | allyl | H | H | COOH |
| 25. | O | O | O | Ph | H | Ph | H | H | COOH |
| 26. | O | O | O | Ph | H | 4-HO-Ph | H | H | COOH |
| 27. | O | O | O | Ph | H | 4-O₂N-Ph | H | H | COOH |
| 28. | O | O | O | Ph | H | 4-MeO₂C-Ph | H | H | COOH |
| 29. | O | O | O | Ph | H | 4-Me-Ph | H | H | COOH |
| 30. | O | O | O | Ph | H | 4-MeO-Ph | H | H | COOH |
| 31. | O | O | O | Ph | H | 4-Cl-Ph | H | H | COOH |
| 32. | O | O | O | Ph | H | 4-Br-Ph | H | H | COOH |
| 33. | O | O | O | Ph | H | 2-HO-Ph | H | H | COOH |
| 34. | O | O | O | Ph | H | 2-O₂N-Ph | H | H | COOH |
| 35. | O | O | O | Ph | H | 2-MeO₂C-Ph | H | H | COOH |
| 36. | O | O | O | Ph | H | 2-Me-Ph | H | H | COOH |
| 37. | O | O | O | Ph | H | 2-MeO-Ph | H | H | COOH |
| 38. | O | O | O | Ph | H | 2-Cl-Ph | H | H | COOH |
| 39. | O | O | O | Ph | H | 2-Br-Ph | H | H | COOH |
| 40. | O | O | O | Ph | H | 2-naphtyl | H | H | COOH |
| 41. | O | O | O | Ph | H | 2-thiényl | H | H | COOH |
| 42. | O | O | O | Ph | H | 4-biphényl | H | H | COOH |
| 43. | O | O | O | Ph | H | 4-MeO₂C-PhCH₂ | H | H | COOH |
| 44. | O | O | O | Ph | H | 4-Me-PhCH₂ | H | H | COOH |
| 45. | O | O | O | Ph | H | 4-MeOPhCH₂ | H | H | COOH |
| 46. | O | O | O | Ph | H | 4-Cl-PhCH₂ | H | H | COOH |
| 47. | O | O | O | Ph | H | 4-Br-PhCH₂ | H | H | COOH |
| 48. | O | O | O | Ph | H | 2-HO-PhCH₂ | H | H | COOH |
| 49. | O | O | O | Ph | H | 2-O₂N-PhCH₂ | H | H | COOH |
| 50. | O | O | O | Ph | H | 2-MeO₂C-PhCH₂ | H | H | COOH |
| 51. | O | O | O | Ph | H | 2-Me-PhCH₂ | H | H | COOH |
| 52. | O | O | O | Ph | H | 2-MeO-PhCH₂ | H | H | COOH |
| 53. | O | O | O | Ph | H | 2-Cl-PhCH₂ | H | H | COOH |
| 54. | O | O | O | Ph | H | 2-Br-PhCH₂ | H | H | COOH |
| 55. | O | O | O | 4-HO-Ph | H | 4-HO-Ph CH₂ | H | H | COOH |
| 56. | O | O | O | 4-HO-Ph | H | 4-O₂N-PhCH₂ | H | H | COOH |
| 57. | O | O | O | 4-HO-Ph | H | 4-MeO₂C-PhCH₂ | H | H | COOH |
| 58. | O | O | O | 4-HO-Ph | H | 4-Me-PhCH₂ | H | H | COOH |
| 59. | O | O | O | 4-HO-Ph | H | 4-MeOPhCH₂ | H | H | COOH |
| 60. | O | O | O | 4-HO-Ph | H | 4-Cl-PhCH₂ | H | H | COOH |
| 61. | O | O | O | 4-HO-Ph | H | 4-Br-PhCH₂ | H | H | COOH |
| 62. | O | O | O | 4-HO-Ph | H | 2-HO-PhCH₂ | H | H | COOH |
| 63. | O | O | O | 4-HO-Ph | H | 2-O₂N-PhCH₂ | H | H | COOH |
| 64. | O | O | O | 4-HO-Ph | H | 2-MeO₂C-PhCH₂ | H | H | COOH |
| 65. | O | O | O | 4-HO-Ph | H | 2-Me-PhCH₂ | H | H | COOH |
| 66. | O | O | O | 4-HO-Ph | H | 2-MeO-PhCH₂ | H | H | COOH |
| 67. | O | O | O | 4-HO-Ph | H | 2-Cl-PhCH₂ | H | H | COOH |
| 68. | O | O | O | 4-HO-Ph | H | 2-Br-PhCH₂ | H | H | COOH |
| 69. | O | O | O | 4-HO-Ph | H | Me | H | H | COOH |
| 70. | O | O | O | 4-HO-Ph | H | CH₃(CH₂)₂ | H | H | COOH |
| 71. | O | O | O | 4-HO-Ph | H | cyclohexyl | H | H | COOH |
| 72. | O | O | O | 4-HO-Ph | H | allyl | H | H | COOH |
| 73. | O | O | O | Ph | H | HO₂C-CH₂ | H | H | COOH |
| 74. | O | O | O | Ph | H | Bn(HO₂C)CH | H | H | COOH |
| 75. | O | O | O | Ph | H | HOCH₂(HO₂C)CH | H | H | COOH |
| 76. | O | O | O | Ph | H | CH₃(HO)CH(HO₂C)CH | H | H | COOH |
| 77. | O | O | O | Ph | H | MeS(CH₂)₂(HO₂C)CH | H | H | COOH |
| 78. | O | O | O | Ph | H | H₂N(CH₂)₃(HO₂C)CH | H | H | COOH |
| 79. | O | O | O | Ph | H | HO₂CCH₂(HO₂C)CH | H | H | COOH |
| 80. | O | O | O | Ph | H | imidazole-CH₂(HO₂C)CH | H | H | COOH |
| 81. | O | O | O | Ph | H | indole-CH₂(HO₂C)CH | H | H | COOH |
| 82. | O | O | O | 4-HO-Ph | H | HO₂C-CH₂ | H | H | COOH |
| 83. | O | O | O | 4-HO-Ph | H | Me(HO₂C)CH | H | H | COOH |
| 84. | O | O | O | 4-HO-Ph | H | (CH₃)₂CH(HO₂C)CH | H | H | COOH |
| 85. | O | O | O | 4-HO-Ph | H | Bn(HO₂C)CH | H | H | COOH |
| 86. | O | O | O | 4-HO-Ph | H | HOCH₂(HO₂C)CH | H | H | COOH |
| 87. | O | O | O | 4-HO-Ph | H | CH₃(HO)CH(HO₂C)CH | H | H | COOH |
| 88. | O | O | O | 4-HO-Ph | H | MeS(CH₂)₂(HO₂C)CH | H | H | COOH |
| 89. | O | O | O | 4-HO-Ph | H | H₂N(CH₂)₃(HO₂C)CH | H | H | COOH |
| 90. | O | O | O | 4-HO-Ph | H | HO₂CCH₂(HO₂C)CH | H | H | COOH |
| 91. | O | O | O | 4-HO-Ph | H | imidazole-CH₂(HO₂C)CH | H | H | COOH |
| 92. | O | O | O | 4-HO-Ph | H | indole-CH₂(HO₂C)CH | H | H | COOH |
| 93. | O | O | O | Ph | Me | PhCH₂ | H | H | COOH |
| 94. | O | O | O | 4-HO-Ph | Me | PhCH₂ | H | H | COOH |
| 95. | O | O | O | Ph | Bn | PhCH₂ | H | H | COOH |
| 96. | O | O | O | 4-HO-Ph | Bn | PhCH₂ | H | H | COOH |
| 97. | O | O | O | Ph | Me | HO₂C-CH₂ | H | H | COOH |
| 98. | O | O | O | 4-HO-Ph | Me | HO₂C-CH₂ | H | H | COOH |
| 99. | O | O | O | Ph | Bn | HO₂C-CH₂ | H | H | COOH |
| 100. | O | O | O | 4-HO-Ph | Bn | HO₂C-CH₂ | H | H | COOH |
| 101. | O | O | O | Ph | Me | Bn(HO₂C)CH | H | H | COOH |
| 102. | O | O | O | 4-HO-Ph | Me | Bn(HO₂C)CH | H | H | COOH |
| 103. | O | HN | O | Ph | H | PhCH₂ | H | H | CH₃ |
| 104. | O | HN | O | Ph | Me | PhCH₂ | H | H | CH₃ |
| 105. | O | HN | O | Ph | Bn | PhCH₂ | H | H | CH₃ |
| 106. | O | HN | O | 4-OH-Ph | H | PhCH₂ | H | H | CH₃ |
| 107. | O | HN | O | 4-OH-Ph | Me | PhCH₂ | H | H | CH₃ |
| 108. | O | HN | O | 4-OH-Ph | Bn | PhCH₂ | H | H | CH₃ |
| 109. | O | HN | O | Ph | H | Ph | H | H | CH₃ |
| 110. | O | HN | O | Ph | Me | Ph | H | H | CH₃ |
| 111. | O | HN | O | Ph | Bn | Ph | H | H | CH₃ |
| 112. | O | HN | O | 4-OH-Ph | H | Ph | H | H | CH₃ |
| 113. | O | HN | O | 4-OH-Ph | Me | Ph | H | H | CH₃ |
| 114. | O | HN | O | 4-OH-Ph | Bn | Ph | H | H | CH₃ |
| 115. | O | HN | O | Ph | H | CH₃-Ph | H | H | CH₃ |
| 116. | O | HN | O | Ph | Me | CH₃-Ph | H | H | CH₃ |
| 117. | O | HN | O | Ph | Bn | CH₃-Ph | H | H | CH₃ |
| 118. | O | HN | O | 4-OH-Ph | H | CH₃-Ph | H | H | CH₃ |
| 119. | O | HN | O | 4-OH-Ph | Me | CH₃-Ph | H | H | CH₃ |
| 120. | O | HN | O | 4-OH-Ph | Bn | CH₃-Ph | H | H | CH₃ |
| 121. | O | HN | O | Ph | H | 4-MeO-PhCH₂ | H | H | CH₃ |
| 122. | O | HN | O | Ph | Me | 4-MeO-PhCH₂ | H | H | CH₃ |
| 123. | O | HN | O | Ph | Bn | 4-MeO-PhCH₂ | H | H | CH₃ |
| 124. | O | HN | O | 4-OH-Ph | H | 4-MeO-PhCH₂ | H | H | CH₃ |
| 125. | O | HN | O | 4-OH-Ph | Me | 4-MeO-PhCH₂ | H | H | CH₃ |
| 126. | O | HN | O | 4-OH-Ph | Bn | 4-MeO-PhCH₂ | H | H | CH₃ |
| 127. | O | HN | O | Ph | H | CH₃-PhCH₂ | H | H | CH₃ |
| 128. | O | HN | O | Ph | Me | CH₃-PhCH₂ | H | H | CH₃ |
| 129. | O | HN | O | Ph | Bn | CH₃-PhCH₂ | H | H | CH₃ |
| 130. | O | HN | O | 4-OH-Ph | H | CH₃-PhCH₂ | H | H | CH₃ |
| 131. | O | HN | O | 4-OH-Ph | Me | CH₃-PhCH₂ | H | H | CH₃ |
| 132. | O | HN | O | 4-OH-Ph | Bn | CH₃-PhCH₂ | H | H | CH₃ |
| 133. | O | HN | O | Ph | H | Me | H | H | CH₃ |
| 134. | O | HN | O | Ph | H | CH₃(CH₂)₂ | H | H | CH₃ |
| 135. | O | HN | O | Ph | H | cyclohexyl | H | H | CH₃ |
| 136. | O | HN | O | Ph | H | allyl | H | H | CH₃ |
| 137. | O | HN | O | 4-OH-Ph | H | Me | H | H | CH₃ |
| 138. | O | HN | O | 4-OH-Ph | H | CH₃(CH₂)₂ | H | H | CH₃ |
| 139. | O | HN | O | 4-OH-Ph | H | cyclohexyl | H | H | CH₃ |
| 140. | O | HN | O | 4-OH-Ph | H | allyl | H | H | CH₃ |
| 141. | O | HN | O | Ph | H | HO₂C-CH₂ | H | H | CH₃ |
| 142. | O | HN | O | Ph | Me | HO₂C-CH₂ | H | H | CH₃ |
| 143. | O | HN | O | Ph | Bn | HO₂C-CH₂ | H | H | CH₃ |
| 144. | O | HN | O | 4-OH-Ph | H | HO₂C-CH₂ | H | H | CH₃ |
| 145. | O | HN | O | 4-OH-Ph | Me | HO₂C-CH₂ | H | H | CH₃ |
| 146. | O | HN | O | 4-OH-Ph | Bn | HO₂C-CH₂ | H | H | CH₃ |
| 147. | O | HN | O | Ph | H | Bn(HO₂C)CH | H | H | CH₃ |
| 148. | O | HN | O | Ph | Me | Bn(HO₂C)CH | H | H | CH₃ |
| 149. | O | HN | O | Ph | Bn | Bn(HO₂C)CH | H | H | CH₃ |
| 150. | O | HN | O | 4-OH-Ph | H | Bn(HO₂C)CH | H | H | CH₃ |
| 151. | O | HN | O | 4-OH-Ph | Me | Bn(HO₂C)CH | H | H | CH₃ |
| 152. | O | HN | O | 4-OH-Ph | Bn | Bn(HO₂C)CH | H | H | CH₃ |
| 153. | H | O | O | Ph | H | PhCH₂ | H | H | COOH |
| 154. | H | O | O | Ph | Me | PhCH₂ | H | H | COOH |
| 155. | H | O | O | Ph | Bn | PhCH₂ | H | H | COOH |
| 156. | H | O | O | 4-HO-Ph | H | PhCH₂ | H | H | COOH |
| 157. | H | O | O | 4-HO-Ph | Me | PhCH₂ | H | H | COOH |
| 158. | H | O | O | 4-HO-Ph | Bn | PhCH₂ | H | H | COOH |
| 159. | H | O | O | Ph | H | HO₂C-CH₂ | H | H | COOH |
| 160. | H | O | O | Ph | Me | HO₂C-CH₂ | H | H | COOH |
| 161. | H | O | O | Ph | Bn | HO₂C-CH₂ | H | H | COOH |
| 162. | H | O | O | 4-HO-Ph | H | HO₂C-CH₂ | H | H | COOH |
| 163. | H | O | O | 4-HO-Ph | Me | HO₂C-CH₂ | H | H | COOH |
| 164. | H | O | O | 4-HO-Ph | Bn | HO₂C-CH₂ | H | H | COOH |
| 165. | H | O | O | Ph | H | Bn(HO₂C)CH | H | H | COOH |
| 166. | H | O | O | Ph | Me | Bn(HO₂C)CH | H | H | COOH |
| 167. | H | O | O | Ph | Bn | Bn(HO₂C)CH | H | H | COOH |
| 168. | H | O | O | 4-HO-Ph | H | Bn(HO₂C)CH | H | H | COOH |
| 169. | H | O | O | 4-HO-Ph | Me | Bn(HO₂C)CH | H | H | COOH |
| 170. | H | O | O | 4-HO-Ph | Bn | Bn(HO₂C)CH | H | H | COOH |
| 171. | H | HN | O | Ph | H | PhCH₂ | H | H | CH₃ |
| 172. | H | HN | O | Ph | H | 4-MeO-PhCH₂ | H | H | CH₃ |
| 173. | H | HN | O | Ph | Me | PhCH₂ | H | H | CH₃ |
| 174. | H | HN | O | Ph | Bn | PhCH₂ | H | H | CH₃ |
| 175. | H | HN | O | 4-OH-Ph | H | PhCH₂ | H | H | CH₃ |
| 176. | H | HN | O | 4-OH-Ph | Me | PhCH₂ | H | H | CH₃ |
| 177. | H | HN | O | 4-OH-Ph | Bn | PhCH₂ | H | H | CH₃ |
| 178. | H | HN | O | Ph | H | HO₂C-CH₂ | H | H | CH₃ |
| 179. | H | HN | O | Ph | Me | HO₂C-CH₂ | H | H | CH₃ |
| 180. | H | HN | O | Ph | Bn | HO₂C-CH₂ | H | H | CH₃ |
| 181. | H | HN | O | 4-OH-Ph | H | HO₂C-CH₂ | H | H | CH₃ |
| 182. | H | HN | O | 4-OH-Ph | Me | HO₂C-CH₂ | H | H | CH₃ |
| 183. | H | HN | O | 4-OH-Ph | Bn | HO₂C-CH₂ | H | H | CH₃ |
| 184. | H | HN | O | Ph | H | Bn(HO₂C)CH | H | H | CH₃ |
| 185. | H | HN | O | Ph | Me | Bn(HO₂C)CH | H | H | CH₃ |
| 186. | H | HN | O | Ph | Bn | Bn(HO₂C)CH | H | H | CH₃ |
| 187. | H | HN | O | 4-OH-Ph | H | Bn(HO₂C)CH | H | H | CH₃ |
| 188. | H | HN | O | 4-OH-Ph | Me | Bn(HO₂C)CH | H | H | CH₃ |
| 189. | H | HN | O | 4-OH-Ph | Bn | Bn(HO₂C)CH | H | H | CH₃ |
| 190. | H | HN | O | Ph | H | Ph | H | H | CH₃ |
| 191. | H | HN | O | Ph | Me | Ph | H | H | CH₃ |
| 192. | H | HN | O | Ph | Bn | Ph | H | H | CH₃ |
| 193. | H | HN | O | 4-OH-Ph | H | Ph | H | H | CH₃ |
| 194. | H | HN | O | 4-OH-Ph | Me | Ph | H | H | CH₃ |
| 195. | H | HN | O | 4-OH-Ph | Bn | Ph | H | H | CH₃ |
| 196. | H | HN | O | Ph | H | CH₃-Ph | H | H | CH₃ |
| 197. | H | HN | O | Ph | Me | CH₃-Ph | H | H | CH₃ |
| 198. | H | HN | O | Ph | Bn | CH₃-Ph | H | H | CH₃ |
| 199. | H | HN | O | 4-OH-Ph | H | CH₃-Ph | H | H | CH₃ |
| 200. | H | HN | O | 4-OH-Ph | Me | CH₃-Ph | H | H | CH₃ |
| 201. | H | HN | O | 4-OH-Ph | Bn | CH₃-Ph | H | H | CH₃ |
| 202. | H | HN | O | Ph | H | 4-MeO-PhCH₂ | H | H | CH₃ |
| 203. | H | HN | O | Ph | Me | 4-MeO-PhCH₂ | H | H | CH₃ |
| 204. | H | HN | O | Ph | Bn | 4-MeO-PhCH₂ | H | H | CH₃ |
| 205. | H | HN | O | 4-OH-Ph | H | 4-MeO-PhCH₂ | H | H | CH₃ |
| 206. | H | HN | O | 4-OH-Ph | Me | 4-MeO-PhCH₂ | H | H | CH₃ |
| 207. | H | HN | O | 4-OH-Ph | Bn | 4-MeO-PhCH₂ | H | H | CH₃ |
| 208. | H | HN | O | Ph | H | CH₃-PhCH₂ | H | H | CH₃ |
| 209. | H | HN | O | Ph | Me | CH₃-PhCH₂ | H | H | CH₃ |
| 210. | H | HN | O | Ph | Bn | CH₃-PhCH₂ | H | H | CH₃ |
| 211. | H | HN | O | 4-OH-Ph | H | CH₃-PhCH₂ | H | H | CH₃ |
| 212. | H | HN | O | 4-OH-Ph | Me | CH₃-PhCH₂ | H | H | CH₃ |
| 213. | H | HN | O | 4-OH-Ph | Bn | CH₃-PhCH₂ | H | H | CH₃ |
| 214. | H | O | O | Ph | H | PhCH₂ | H | H | CH₂OH |
| 215. | H | O | O | Ph | H | 4-MeOPhCH₂ | H | H | CH₂OH |
| 216. | H | O | O | Ph | Me | PhCH₂ | H | H | CH₂OH |
| 217. | H | O | O | Ph | Bn | PhCH₂ | H | H | CH₂OH |
| 218. | H | O | O | 4-HO-Ph | H | PhCH₂ | H | H | CH₂OH |
| 219. | H | O | O | 4-HO-Ph | Me | PhCH₂ | H | H | CH₂OH |
| 220. | H | O | O | 4-HO-Ph | Bn | PhCH₂ | H | H | CH₂OH |
| 221. | H | O | O | Ph | H | HOCH₂ | H | H | CH₂OH |
| 222. | H | O | O | Ph | Me | HOCH₂ | H | H | CH₂OH |
| 223. | H | O | O | Ph | Bn | HOCH₂ | H | H | CH₂OH |
| 224. | H | O | O | 4-HO-Ph | H | HOCH₂ | H | H | CH₂OH |
| 225. | H | O | O | 4-HO-Ph | Me | HOCH₂ | H | H | CH₂OH |
| 226. | H | O | O | 4-HO-Ph | Bn | HOCH₂ | H | H | CH₂OH |
| 227. | H | O | O | Ph | H | Bn(HOH₂C)CH | H | H | CH₂OH |
| 228. | H | O | O | Ph | Me | Bn(HOH₂C)CH | H | H | CH₂OH |
| 229. | H | O | O | Ph | Bn | Bn(HOH₂C)CH | H | H | CH₂OH |
| 230. | H | O | O | 4-HO-Ph | H | Bn(HOH₂C)CH | H | H | CH₂OH |
| 231. | H | O | O | 4-HO-Ph | Me | Bn(HOH₂C)CH | H | H | CH₂OH |
| 232. | H | O | O | 4-HO-Ph | Bn | Bn(HOH₂C)CH | H | H | CH₂OH |
| 233. | H | HN | O | Ph | H | PhCH₂ | H | H | H |
| 234. | H | HN | O | Ph | H | 4-MeO-PhCH₂ | H | H | H |
| 235. | H | HN | O | Ph | Me | PhCH₂ | H | H | H |
| 236. | H | HN | O | Ph | Bn | PhCH₂ | H | H | H |
| 237. | H | HN | O | 4-OH-Ph | H | PhCH₂ | H | H | H |
| 238. | H | HN | O | 4-OH-Ph | Me | PhCH₂ | H | H | H |
| 239. | H | HN | O | 4-OH-Ph | Bn | PhCH₂ | H | H | H |
| 240. | H | HN | O | Ph | H | HOCH₂ | H | H | H |
| 241. | H | HN | O | Ph | Me | HOCH₂ | H | H | H |
| 242. | H | HN | O | Ph | Bn | HOCH₂ | H | H | H |
| 243. | H | HN | O | 4-OH-Ph | H | HOCH₂ | H | H | H |
| 244. | H | HN | O | 4-OH-Ph | Me | HOCH₂ | H | H | H |
| 245. | H | HN | O | 4-OH-Ph | Bn | HOCH₂ | H | H | H |
| 246. | H | HN | O | Ph | H | Bn(HOH₂C)CH | H | H | H |
| 247. | H | HN | O | Ph | Me | Bn(HOH₂C)CH | H | H | H |
| 248. | H | HN | O | Ph | Bn | Bn(HOH₂C)CH | H | H | H |
| 249. | H | HN | O | 4-OH-Ph | H | Bn(HOH₂C)CH | H | H | H |
| 250. | H | HN | O | 4-OH-Ph | Me | Bn(HOH₂C)CH | H | H | H |
| 251. | H | HN | S | Ph | H | PhCH₂ | H | H | H |
| 252. | H | HN | S | Ph | H | 4-MeO-PhCH₂ | H | H | H |
| 253. | H | HN | S | Ph | Me | PhCH₂ | H | H | H |
| 254. | H | HN | S | Ph | Bn | PhCH₂ | H | H | H |
| 255. | H | HN | S | 4-OH-Ph | H | PhCH₂ | H | H | H |
| 256. | H | HN | S | 4-OH-Ph | Me | PhCH₂ | H | H | H |
| 257. | H | HN | S | 4-OH-Ph | Bn | PhCH₂ | H | H | H |
| 258. | H | HN | S | Ph | H | HOCH₂ | H | H | H |
| 259. | H | HN | S | Ph | Me | HOCH₂ | H | H | H |
| 260. | H | HN | S | Ph | Bn | HOCH₂ | H | H | H |
| 261. | H | HN | S | 4-OH-Ph | H | HOCH₂ | H | H | H |
| 262. | H | HN | S | 4-OH-Ph | Me | HOCH₂ | H | H | H |
| 263. | H | HN | S | 4-OH-Ph | Bn | HOCH₂ | H | H | H |
| 264. | H | HN | S | Ph | H | Bn(HOH₂C)CH | H | H | H |
| 265. | H | HN | S | Ph | Me | Bn(HOH₂C)CH | H | H | H |
| 266. | H | HN | S | Ph | Bn | Bn(HOH₂C)CH | H | H | H |
| 267. | H | HN | S | 4-OH-Ph | H | Bn(HOH₂C)CH | H | H | H |
| 268. | H | HN | S | 4-OH-Ph | Me | Bn(HOH₂C)CH | H | H | H |

6. Procédé de préparation de composés de formule (I) selon la revendication 1, dans lequel un composé de formule (II) 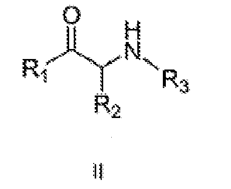 dans laquelle R₁, R₂ et R₃ sont comme défini ci-dessus
réagit avec un composé de formule (III) 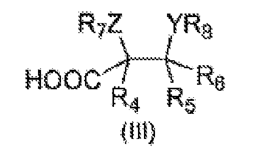 dans laquelle R₄, R₅, R₆, Y et Z comme défini ci-dessus et R₇ et R₈ représentent H ou des groupes protecteurs appropriés, (Pg), qui peuvent être identiques ou différents, cycliques or acycliques, et qui peuvent être clivés dans des conditions acides, afin de donner un composé de formule (IV) 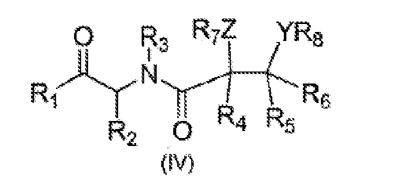 dans laquelle les substituants ont la signification donnée ci-dessus, qui est cyclisé pour donner un composé de formule (I) par action d'un acide.

7. Procédé selon la revendication 6, dans lequel la première étape est effectuée dans un solvant polaire aprotique à une température comprise entre 0 et 100 °C pendant 1 à 24 heures.

8. Procédé selon la revendication 7, dans lequel la réaction est effectuée en présence d'un agent de couplage.

9. Procédé selon la revendication 6, dans lequel la seconde étape est effectuée en présence d'un acide fort à une température de 0 à 150 °C pendant 15 min à 24 heures.

10. Procédé selon la revendication 9, dans lequel l'acide est choisi dans le groupe constitué par l'acide sulfurique adsorbé sur gel de silice, l'acide p-toluène sulfonique, l'acide trifluoroacétique et l'acide trifluorométhanesulfonique.
